(19) Europäisches Patentamt
European Patent Office
Office européen des brevets

(11) EP 4 670 719 A1

(12) # EUROPEAN PATENT APPLICATION
published in accordance with Art. 153(4) EPC

(43) Date of publication:
**31.12.2025 Bulletin 2026/01**

(21) Application number: **24759705.7**

(22) Date of filing: **21.02.2024**

(51) International Patent Classification (IPC):
***A61K 31/454*** *(2006.01)* ***C07D 401/02*** *(2006.01)*
***C07D 401/06*** *(2006.01)* ***C07D 403/02*** *(2006.01)*
***C07D 403/06*** *(2006.01)* ***A61K 9/00*** *(2006.01)*
***A61K 47/00*** *(2006.01)* ***A61P 13/12*** *(2006.01)*

(52) Cooperative Patent Classification (CPC):
**A61K 9/00; A61K 31/454; A61K 47/00; A61P 13/12; C07D 401/02; C07D 401/06; C07D 403/02; C07D 403/06**

(86) International application number:
**PCT/CN2024/077932**

(87) International publication number:
**WO 2024/175028 (29.08.2024 Gazette 2024/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **22.02.2023 CN 202310151026**

(71) Applicant: **Haisco Pharmaceutical Group Co., Ltd.**
**Shannan, Tibet 856000 (CN)**

(72) Inventors:
- **ZHANG, Xuanmiao**
  **Lhoka, Tibet 856099 (CN)**
- **DOU, Ying**
  **Lhoka, Tibet 856099 (CN)**
- **MAO, Hua**
  **Lhoka, Tibet 856099 (CN)**
- **DENG, Shihao**
  **Lhoka, Tibet 856099 (CN)**
- **ZHOU, Yang**
  **Lhoka, Tibet 856099 (CN)**

(74) Representative: **AWA Denmark A/S**
**Strandgade 56**
**1401 Copenhagen K (DK)**

(54) **PHARMACEUTICAL COMPOSITION OF BENZO NITROGEN HETEROAROMATIC RING DERIVATIVE AND USE THEREOF IN MEDICINE**

(57) A pharmaceutical composition of a benzo nitrogen heteroaromatic ring derivative and the use thereof in medicine. The pharmaceutical composition contains active ingredient A and a pharmaceutical excipient, wherein the active ingredient A is selected from a compound as represented by general formula (I) or a stereoisomer, tautomer, deuterated compound, solvate, prodrug, metabolite, pharmaceutically acceptable salt or cocrystal thereof. The pharmaceutical composition contains 1-1000 mg of the active ingredient A, and the content of the active ingredient A is selected from 1.0-90.0%. The present invention also relates to the use of the pharmaceutical composition in the preparation of a related drug for treating nephropathy.

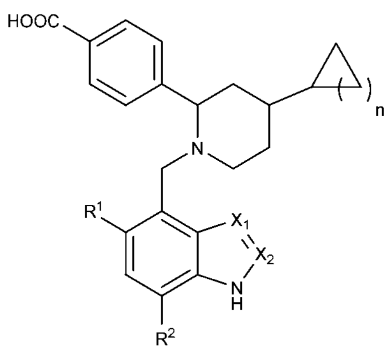

(I)

EP 4 670 719 A1

## Description

### Technical Field

The present invention belongs to the field of pharmaceutical formulations, and in particular relates to a pharmaceutical composition, which comprises a therapeutically effective amount of an active ingredient A and a pharmaceutically acceptable excipient, wherein the active ingredient A is selected from a compound represented by general formula (I) or a stereoisomer, a tautomer, a deuterated form, a solvate, a prodrug, a metabolite, a pharmaceutically acceptable salt or an eutectic crystal thereof; and the pharmaceutical composition comprises 1-1000 mg of the active ingredient A, which is present in a content of 1.0%-90.0%. The present invention further relates to the use of the pharmaceutical composition in the preparation of a related drug for treating nephropathy.

### Background Art

Complement factor B is a component of the complement alternative pathway and participates in the body's specific and non-specific immune mechanisms. It contains a serine protease (SP) domain. When activated, it will provide catalytic activity of C3 and C5 convertases of the alternative pathway. Complement factor B is a key enzyme in the activation process of the complement alternative pathway and can be used as a suitable target to inhibit the complement activation pathway.

Patent PCT/CN 2022/113216 recites compound 1, which has good inhibitory activity against complement factor B and a high in-vivo C3a inhibition rate. When the compound isused as a therapeutic agent for the treatment of humans, the administration of same at therapeutically effective doses is problematic and can result in toxic side effects or therapeutic ineffectiveness. Therefore, it is essential to develop a pharmaceutical composition or a pharmaceutical formulation with good safety, effectiveness and stability.

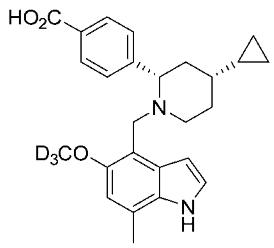

(Compound 1)

### Summary of the Invention

The objective of the present invention relates to a pharmaceutical composition, which comprises an active ingredient A and a pharmaceutically acceptable excipient, wherein the active ingredient A is selected from a compound represented by general formula (I) or a stereoisomer, a tautomer, a deuterated form, solvate, a prodrug, a metabolite, a pharmaceutically acceptable salt or an eutectic crystal thereof. The present invention further relates to the use of the pharmaceutical composition in the preparation of a related drug for treating nephropathy.

The pharmaceutical composition of the present invention has stable quality, a high dissolution rate, good absorption, low irritation, good safety and high bioavailability, and is orally absorbable.

The present invention relates to a pharmaceutical composition, which comprises a therapeutically effective amount of an active ingredient A and a pharmaceutically acceptable excipient. The pharmaceutical composition can be in a unit formulation form.

The present invention relates to a pharmaceutical composition, which comprises an active ingredient A and a pharmaceutically acceptable excipient, wherein the active ingredient A is selected from a compound represented by general formula (I) or a stereoisomer, a tautomer, a deuterated form, a solvate, a prodrug, a metabolite, a pharmaceutically acceptable salt or an eutectic crystal thereof,

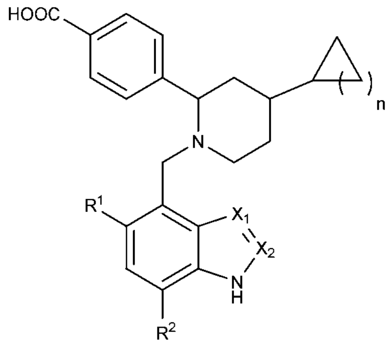

(I)

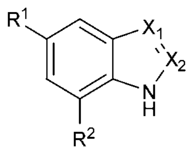

is selected from

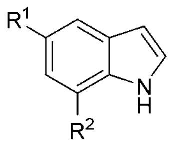 , 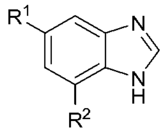 , or 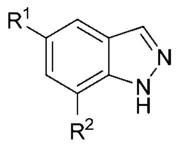 ;

$R^1$ is selected from $-OCH_3$ or $-OCD_3$; $R^2$ is selected from $-CH_3$ or $-CD_3$; n is selected from 1, 2 or 3; and the content of the active ingredient A is selected from 1.0%-90.0%, preferably 5.0%-75.0%, more preferably 10.0%-50.0%.

In some embodiments, the structure of the compound represented by general formula (I) is selected from one of the structures shown in Table S-1.

Table S-1 Compound structures

| | | |
|---|---|---|
| 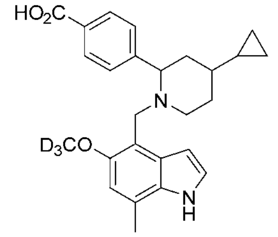 | 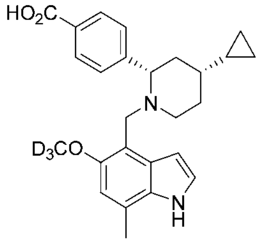 | 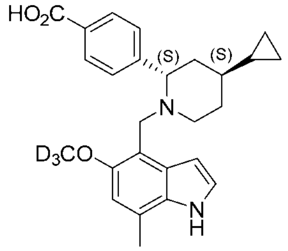 |
| 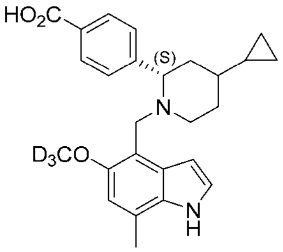 | 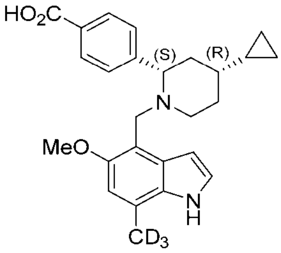 | 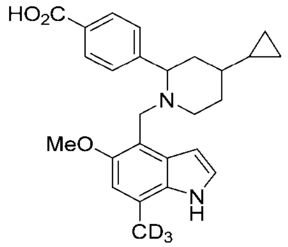 |
| 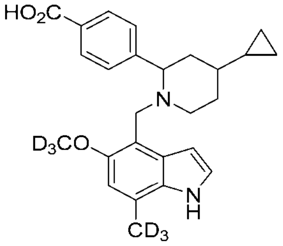 | 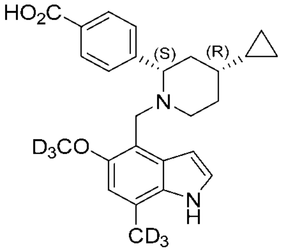 | 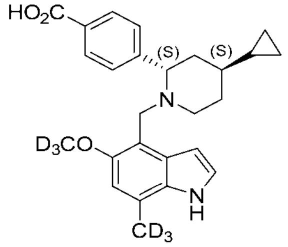 |

| | | |
|---|---|---|
| 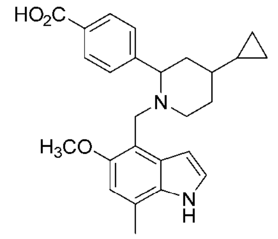 | 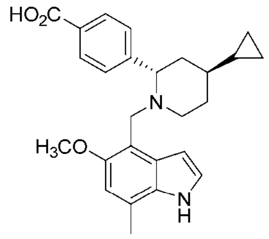 | 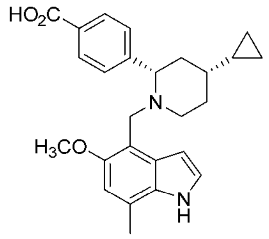 |
| 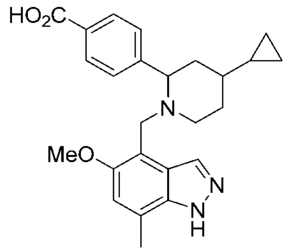 | 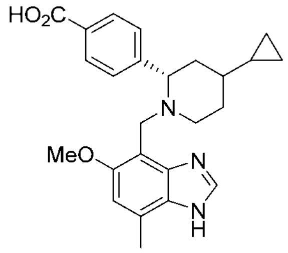 | 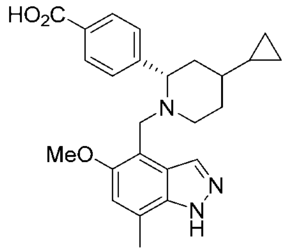 |
| 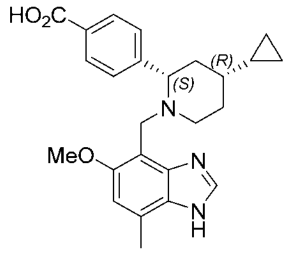 | 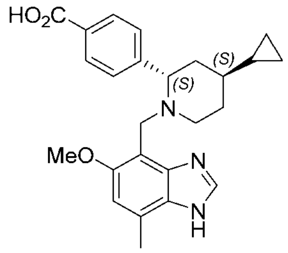 | 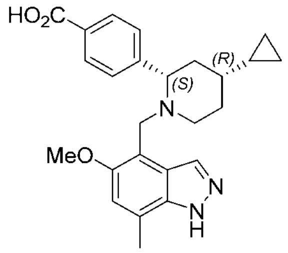 |
| 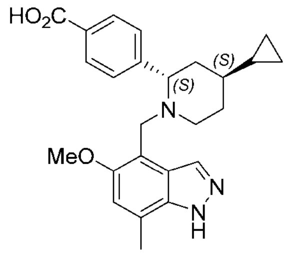 | 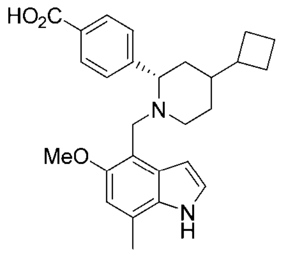 | 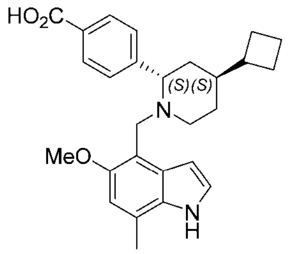 |
| 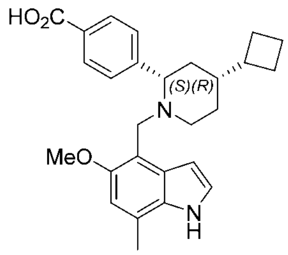 | 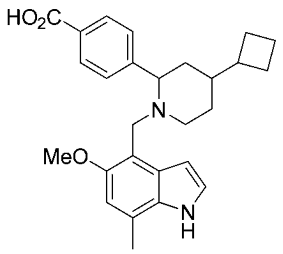 | 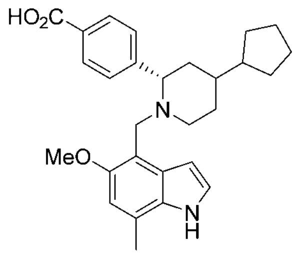 |
| 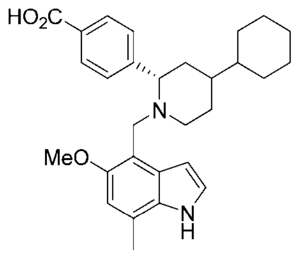 | 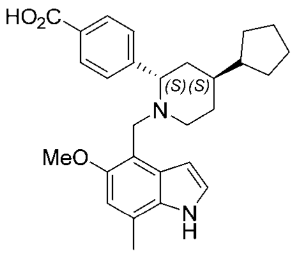 | 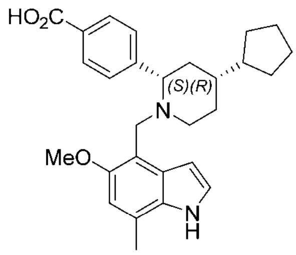 |

.

In some embodiments, the compound represented by general formula (I) is selected from compound 1, which has the following structure:

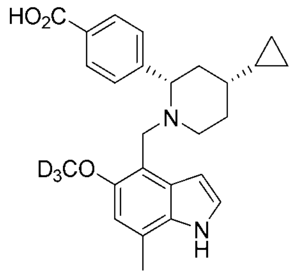

(Compound 1).

In some embodiments, the pharmaceutically acceptable salt of the compound represented by general formula (I) is selected from trifluoroacetate or succinate, and further the molar ratio of compound 1 to the succinate is 1 : 0.75.

In one aspect, the pharmaceutical composition comprises 1-1000 mg of the active ingredient A (calculated based on a free base);

in some embodiments, the pharmaceutical composition comprises 5-900 mg of the active ingredient A (calculated based on a free base);
in some embodiments, the pharmaceutical composition comprises 10-800 mg of the active ingredient A (calculated based on a free base);
in some embodiments, the pharmaceutical composition comprises 10-400 mg of the active ingredient A (calculated based on a free base);
in some embodiments, the pharmaceutical composition comprises 20-400 mg of the active ingredient A (calculated based on a free base);
in some embodiments, the pharmaceutical composition comprises 30-300 mg of the active ingredient A (calculated based on a free base);
in some embodiments, the pharmaceutical composition comprises 20-200 mg of the active ingredient A (calculated based on a free base);
in some embodiments, the pharmaceutical composition comprises 30-200 mg of the active ingredient A (calculated based on a free base);
in some embodiments, the pharmaceutical composition comprises 30-100 mg of the active ingredient A (calculated based on a free base);
in some embodiments, the pharmaceutical composition comprises 50-100 mg of the active ingredient A (calculated based on a free base);
in some embodiments, the pharmaceutical composition comprises 5-100 mg of the active ingredient A (calculated based on a free base);
in some embodiments, the pharmaceutical composition comprises 5 mg of the active ingredient A (calculated based on a free base);
in some embodiments, the pharmaceutical composition comprises 10 mg of the active ingredient A (calculated based on a free base);
in some embodiments, the pharmaceutical composition comprises 20 mg of the active ingredient A (calculated based on a free base);
in some embodiments, the pharmaceutical composition comprises 25 mg of the active ingredient A (calculated based on a free base);
in some embodiments, the pharmaceutical composition comprises 30 mg of the active ingredient A (calculated based on a free base);
in some embodiments, the pharmaceutical composition comprises 40 mg of the active ingredient A (calculated based on a free base);
in some embodiments, the pharmaceutical composition comprises 50 mg of the active ingredient A (calculated based on a free base);
in some embodiments, the pharmaceutical composition comprises 75 mg of the active ingredient A (calculated based on a free base);
in some embodiments, the pharmaceutical composition comprises 100 mg of the active ingredient A (calculated based on a free base);
in some embodiments, the pharmaceutical composition comprises 125 mg of the active ingredient A (calculated based on a free base);
in some embodiments, the pharmaceutical composition comprises 150 mg of the active ingredient A (calculated based on a free base);
in some embodiments, the pharmaceutical composition comprises 200 mg of the active ingredient A (calculated

based on a free base);

in some embodiments, the pharmaceutical composition comprises 300 mg of the active ingredient A (calculated based on a free base);

in some embodiments, the pharmaceutical composition comprises 400 mg of the active ingredient A (calculated based on a free base);

in some embodiments, the pharmaceutical composition comprises 500 mg of the active ingredient A (calculated based on a free base);

in some embodiments, the pharmaceutical composition comprises 600 mg of the active ingredient A (calculated based on a free base);

in some embodiments, the pharmaceutical composition comprises 700 mg of the active ingredient A (calculated based on a free base); and

in some embodiments, the pharmaceutical composition comprises 800 mg of the active ingredient A (calculated based on a free base).

In another aspect, the content of the active ingredient A in the aforementioned pharmaceutical composition is selected from 1.0%-90.0%, preferably 5.0%-75.0%, more preferably 10.0%-50.0%.

In some embodiments, the active ingredient A in the aforementioned pharmaceutical composition is selected from compound 1 or succinate thereof.

In some embodiments, the active ingredient A in the aforementioned pharmaceutical composition is succinate of compound 1; and preferably, the molar ratio of compound 1 to succinic acid is 1 : 0.75.

In some embodiments, the content of the active ingredient A is 1.0%-90.0%; in some embodiments, the content of the active ingredient A is 1.0%-80.0%; in some embodiments, the content of the active ingredient A is 1.0%-70.0%; in some embodiments, the content of the active ingredient A is 1.0%-60.0%; in some embodiments, the content of the active ingredient A is 1.0%-50.0%; in some embodiments, the content of the active ingredient A is 1.0%-40.0%; in some embodiments, the content of the active ingredient A is 1.0%-30.0%; in some embodiments, the content thereof is 1.0%-20.0%; in some embodiments, the content of the active ingredient A is 1.0%-10.0%; in some embodiments, the content of the active ingredient A is 5.0%-90.0%; in some embodiments, the content of the active ingredient A is 5.0%-80.0%; in some embodiments, the content of the active ingredient A is 5.0%-70.0%; in some embodiments, the content of the active ingredient A is 5.0%-60.0%; in some embodiments, the content of the active ingredient A is 5.0%-50.0%; in some embodiments, the content of the active ingredient A is 5.0%-40.0%; in some embodiments, the content of the active ingredient A is 5.0%-30.0%; in some embodiments, the content of the active ingredient A is 5.0%-20.0%; in some embodiments, the content of the active ingredient A is 5.0%-10.0%; in some embodiments, the content of the active ingredient A is 10.0%-90.0%; in some embodiments, the content of the active ingredient A is 10.0%-80.0%; in some embodiments, the content of the active ingredient A is 10.0%-70.0%; in some embodiments, the content of the active ingredient A is 10.0%-60.0%; in some embodiments, the content of the active ingredient A is 10.0%-50.0%; in some embodiments, the content of the active ingredient A is 10.0%-40.0%; in some embodiments, the content of the active ingredient A is 10.0%-30.0%; in some embodiments, the content of the active ingredient A is 10.0%-20.0%; and in some embodiments, the content of the active ingredient A is 10.0%.

In yet another aspect, the pharmaceutically acceptable excipient in the aforementioned pharmaceutical composition is selected from one or more of a filler, a disintegrant, a binder, a glidant and a lubricant.

In some embodiments, the filler is selected from one or more of microcrystalline cellulose, mannitol, lactose, sucrose, sorbitol, dextran, pregelatinized starch, calcium dihydrogen phosphate and starch.

In some embodiments, the content of the filler is selected from 10.0%-90.0%, preferably 20.0%-80.0%;

in some embodiments, the content of the filler is 10.0%-90.0%; in some embodiments, the content thereof is 20.0%-90.0%; in some embodiments, the content thereof is 30.0%-90.0%; in some embodiments, the content thereof is 40.0%-90.0%; in some embodiments, the content thereof is 50.0%-90.0%; in some embodiments, the content thereof is 60.0%-90.0%; in some embodiments, the content thereof is 70.0%-90.0%; in some embodiments, the content thereof is 10.0%-80.0%; in some embodiments, the content thereof is 20.0%-80.0%; in some embodiments, the content thereof is 30.0%-80.0%; in some embodiments, the content thereof is 40.0%-80.0%; in some embodiments, the content thereof is 50.0%-80.0%; in some embodiments, the content thereof is 60.0%-80.0%; in some embodiments, the content thereof is 70.0%-80.0%.

In some embodiments, the filler is selected from a combination of microcrystalline cellulose and lactose, and the content thereof is selected from 10%-90%, preferably 20%-80%, wherein the content ratio of microcrystalline cellulose to lactose is 2 : 1 to 1 : 3, preferably 1 : 1 to 1 : 2.

In some embodiments, the filler is selected from a combination of microcrystalline cellulose and lactose, and the content ratio of microcrystalline cellulose to lactose is 1 : 1 to 1 : 3; in some embodiments, the content ratio of microcrystalline cellulose to lactose is 1 : 1 to 1 : 2; in some embodiments, the content ratio of microcrystalline cellulose to lactose is 1 : 1 to 1 : 2.5; and in some embodiments, the content ratio of microcrystalline cellulose to lactose is 1 : 1 to 1 : 1.5.

In some embodiments, the filler is selected from a combination of microcrystalline cellulose and lactose, and the content of microcrystalline cellulose accounts for 15.0%-80.0% of the total content; in some embodiments, the content of microcrystalline cellulose accounts for 30.0%-80.0% of the total content; in some embodiments, the content of microcrystalline cellulose accounts for 20.0% of the total content; in some embodiments, the content of lactose accounts for 30.0%-60.0% of the total content; in some embodiments, the content of lactose accounts for 15.0%-60.0% of the total content; and in some embodiments, the content of lactose accounts for 40.0%-55.0% of the total content.

In some embodiments, the disintegrant is selected from one or more of sodium carboxymethyl starch, low-substituted hydroxypropyl cellulose, crospovidone, croscarmellose sodium and calcium carboxymethylcellulose, and the content thereof is selected from 1.0%-10.0%, preferably 3.0%-8.0%.

In some embodiments, the disintegrant is selected from sodium carboxymethyl starch, and the content thereof accounts for 1.0%-8.0% of the total content; in some embodiments, the content thereof accounts for 3.0%-8.0% of the total content; in some embodiments, the content thereof accounts for 3.0%-5.0% of the total content; and in some embodiments, the content thereof accounts for 3.0% of the total content.

In some embodiments, the binder is selected from one or more of povidone, hydroxypropyl cellulose, hypromellose and methylcellulose, and the content thereof is selected from 1.0%-10.0%, preferably 2.0%-8.0%.

In some embodiments, the binder is selected from copovidone, and the content thereof accounts for 1.0%-8.0% of the total content; in some embodiments, the content thereof accounts for 3.0%-8.0% of the total content; in some embodiments, the content thereof accounts for 3.0%-5.0% of the total content; and in some embodiments, the content thereof accounts for 3.0% of the total content.

In some embodiments, the wetting agent is selected from one or both of water and ethanol.

In some embodiments, the glidant is selected from one or more of talc, silica, colloidal silicon dioxide, polyethylene glycol and magnesium lauryl sulfate, and the content thereof is selected from 0.1%-3.0%.

In some embodiments, the glidant is selected from silica, and the content thereof accounts for 0.1%-3.0% of the total content; in some embodiments, the content thereof accounts for 0.1%-2.0% of the total content; in some embodiments, the content thereof accounts for 0.1%-1.0% of the total content; in some embodiments, the content thereof accounts for 0.5%-2.0% of the total content; in some embodiments, the content thereof accounts for 0.5%-1.0% of the total content; in some embodiments, the content thereof accounts for 1.0%-3.0% of the total content; and in some embodiments, the content thereof accounts for 1.0% of the total content.

In some embodiments, the lubricant is selected from one or more of magnesium stearate, calcium stearate, stearic acid and sodium stearyl fumarate, and the content thereof is selected from 0.1%-3.0%.

In some embodiments, the lubricant is selected from sodium stearyl fumarate, and the content thereof accounts for 0.1%-3.0% of the total content; in some embodiments, the content thereof accounts for 0.1%-2.0% of the total content; in some embodiments, the content thereof accounts for 0.1%-1.0% of the total content; in some embodiments, the content thereof accounts for 0.5%-2.0% of the total content; in some embodiments, the content thereof accounts for 0.5%-1.0% of the total content; in some embodiments, the content thereof accounts for 1.0%-3.0% of the total content; and in some embodiments, the content thereof accounts for 1.0% of the total content.

In some embodiments, the pharmaceutically acceptable excipient further comprises one or more of a flavoring agent, an antioxidant, a preservative, an opacifier and a film coating premixer.

The pharmaceutical composition of any one of embodiments of the present invention comprises the active ingredient A of any one of the preceding embodiments, wherein the active ingredient A is preferably compound 1 or succinate or crystal form thereof; the pharmaceutically acceptable excipient comprises a filler and a disintegrant, and preferably, further comprises one or more of a binder, a glidant, a lubricant and a pH regulator; and the pharmaceutical composition comprises:

(i) the active ingredient A, and the content thereof is 1.0%-90.0% ; in some embodiments, the content thereof is 1.0%-80.0%; in some embodiments, the content thereof is 1.0%-70.0%; in some embodiments, the content thereof is 1.0%-60.0%; in some embodiments, the content thereof is 1.0%-50.0%; in some embodiments, the content thereof is 1.0%-40.0%; in some embodiments, the content thereof is 1.0%-30.0%; in some embodiments, the content thereof is 1.0%-20.0%; in some embodiments, the content thereof is 1.0%-10.0%; in some embodiments, the content thereof is 5.0%-90.0%; in some embodiments, the content thereof is 5.0%-80.0%; in some embodiments, the content thereof is 5.0%-70.0%; in some embodiments, the content thereof is 5.0%-60.0%; in some embodiments, the content thereof is 5.0%-50.0%; in some embodiments, the content thereof is 5.0%-40.0%; in some embodiments, the content thereof is 5.0%-30.0%; in some embodiments, the content thereof is 5.0%-20.0%; in some embodiments, the content thereof is 5.0%-10.0%; in some embodiments, the content thereof is 10.0%-90.0%; in some embodiments, the content thereof is 10.0%-80.0%; in some embodiments, the content thereof is 10.0%-70.0%; in some embodiments, the content thereof is 10.0%-60.0%; in some embodiments, the content thereof is 10.0%-50.0%; in some embodiments, the content thereof is 10.0%-40.0%; in some embodiments, the content thereof is 10.0%-30.0%; in some embodiments, the content thereof is 10.0%-20.0%; and in some embodiments, the content thereof is 10.0%;

(ii) the filler, which is a combination of microcrystalline cellulose and lactose, and the content thereof is 50.0%-90.0%; in some embodiments, the content thereof is 60.0%-90.0%; in some embodiments, the content thereof is 70.0%-90.0%; in some embodiments, the content thereof is 50.0%-80.0%; in some embodiments, the content thereof is 60.0%-80.0%; in some embodiments, the content thereof is 70.0%-85.0%; in some embodiments, the content thereof is 75.0%-82.0%; further, in some embodiments, the content ratio of microcrystalline cellulose to lactose is 1 : 1 to 1 : 2; and in some embodiments, the content ratio of microcrystalline cellulose to lactose is 1 : 1 to 1 : 1.5;
(iii) the disintegrant, which is croscarmellose sodium, and the content thereof is 1.0%-8.0%; in some embodiments, the content thereof accounts for 1%-3% of the total content; in some embodiments, the content thereof accounts for 3.0%-8.0% of the total content; and in some embodiments, the content thereof accounts for 3% of the total content;
(iv) the binder, which is copovidone, and the content thereof is 1.0%-8.0%; in some embodiments, the content thereof accounts for 1.0%-3.0% of the total content; in some embodiments, the content thereof accounts for 3.0%-8.0% of the total content; and in some embodiments, the content thereof accounts for 3.0% of the total content;
(v) the lubricant, which is sodium stearyl fumarate, and the content thereof is 0.1%-3.0%; in some embodiments, the content thereof accounts for 0.1%-2.0% of the total content; in some embodiments, the content thereof accounts for 0.1%-1.0% of the total content; in some embodiments, the content thereof accounts for 0.5%-2.0% of the total content; in some embodiments, the content thereof accounts for 0.5%-1.0% of the total content; in some embodiments, the content thereof accounts for 1.0%-3.0% of the total content; and in some embodiments, the content thereof accounts for 1.0% of the total content;
(vi) the glidant, which is silica, and the content thereof is 0.1%-3.0%; in some embodiments, the content thereof accounts for 0.1%-2.0% of the total content; in some embodiments, the content thereof accounts for 0.1%-1.0% of the total content; in some embodiments, the content thereof accounts for 0.5%-2.0% of the total content; in some embodiments, the content thereof accounts for 0.5%-1.0% of the total content; in some embodiments, the content thereof accounts for 1.0%-3.0% of the total content; and in some embodiments, the content thereof accounts for 1.0% of the total content.

The pharmaceutical composition of any one of embodiments of the present invention comprises:

(i) compound 1 or succinate thereof, the content thereof is 1.0%-90.0%; and optionally,
(ii) a pharmaceutically acceptable excipient, which includes one or more of a filler, a binder, a wetting agent, a disintegrant, a glidant and a lubricant.
(iii) a filler, which includes one or more of microcrystalline cellulose, mannitol, lactose, sucrose, sorbitol, dextran, pregelatinized starch, calcium dihydrogen phosphate and starch;
(iv) a binder, which includes one or more of povidone, hydroxypropyl cellulose, hypromellose and methylcellulose;
(v) a wetting agent, which includes one or both of water and ethanol;
(vi) a disintegrant, which includes one or more of sodium carboxymethyl starch, low-substituted hydroxypropyl cellulose, crospovidone, croscarmellose sodium and calcium carboxymethylcellulose;
(vii) a glidant, which includes one or more of talc, silica, colloidal silicon dioxide, polyethylene glycol and dodecyl magnesium sulfate;
(viii) a lubricant, which includes magnesium stearate, calcium stearate, stearic acid and sodium stearyl fumarate; and
(ix) one or more of a flavoring agent, an antioxidant, a preservative, an opacifier and a film coating premixer may be further included.

Optionally, the pharmaceutical composition of any one of the aforementioned embodiments can be prepared into a formulation in the form of a tablet, a granule, a powder, an oral solution, an emulsion or a capsule.
Optionally, for the pharmaceutical formulation of any one of the aforementioned embodiments, the binder can be added in a solution or powder form; and the disintegrant can be added internally, externally, or both.
The present invention further provides the use of the pharmaceutical composition in the preparation of a related drug for treating nephropathy.
Unless stated to the contrary, the terms used in the description and claims of the present application have the following meanings.
The term "formulation specification" refers to the weight of the active drug (active ingredient A) contained in each vial, tablet, or other unit formulation.
The carbon, hydrogen, oxygen, sulfur, nitrogen, F, Cl, Br and I involved in the groups and compounds of the present invention all comprise their isotopes, and the carbon, hydrogen, oxygen, sulfur or nitrogen involved in the groups and compounds of the present invention is optionally further substituted with one or more of their corresponding isotopes, wherein the isotopes of carbon comprise $^{12}C$, $^{13}C$ and $^{14}C$, the isotopes of hydrogen comprise protium (H), deuterium (D, also known as heavy hydrogen) and tritium (T, also known as superheavy hydrogen), the isotopes of oxygen comprise $^{16}O$,

$^{17}O$ and $^{18}O$, the isotopes of sulfur comprise $^{32}S$, $^{33}S$, $^{34}S$ and $^{36}S$, the isotopes of nitrogen comprise $^{14}N$ and $^{15}N$, the isotopes of fluorine comprise $^{17}F$ and $^{19}F$, the isotopes of chlorine comprise $^{35}Cl$ and $^{37}Cl$, and the isotopes of bromine comprise $^{79}Br$ and $^{81}Br$.

The term "optional" or "optionally" refers to that the events or circumstances subsequently described may but not necessarily occur, and the description includes the occasions where the events or circumstances occur or do not occur. For example, the term "alkyl optionally substituted with F" means that the alkyl may but not necessarily be substituted with F, and the description includes the case where the alkyl is substituted with F and the case where the alkyl is not substituted with F.

The term "pharmaceutically acceptable salt" or "pharmaceutically acceptable salt thereof" refers to a salt of the compound according to the present invention, which salt maintains the biological effectiveness and characteristics of a free acid or a free base, and is obtained by reacting the free acid with a non-toxic inorganic base or organic base, or reacting the free base with a non-toxic inorganic acid or organic acid.

The term "pharmaceutical composition" refers to a mixture of one or more compounds of the present invention, or stereoisomers, tautomers, deuterated forms, solvates, prodrugs, metabolites, pharmaceutically acceptable salts or eutectic crystals thereof and other chemical components, wherein the term "other chemical components" refer to pharmaceutically acceptable carriers, excipients and/or one or more other therapeutic agents.

The term "carrier" refers to a material that does not cause significant irritation to an organism and does not eliminate the biological activity and characteristics of a compound administered.

The term "excipient" refers to an inert substance added to a pharmaceutical composition to facilitate the administration of a compound. Non-limiting examples include calcium carbonate, calcium phosphate, sugar, starch, cellulose derivatives (including microcrystalline cellulose), gelatin, vegetable oils, polyethylene glycols, diluents, granulating agents, lubricants, binders and disintegrants.

The term "prodrug" refers to a compound that can be converted into the compound of the present invention with the biological activity by means of metabolism in vivo. The prodrug of the present invention is prepared by modifying an amino or carboxyl group in the compound of the present invention, and the modification can be removed by conventional operations or removed in vivo to obtain a parent compound. When the prodrug of the present invention is administered to a mammalian individual, the prodrug is split to form a free amino or carboxyl group.

The term "eutectic crystal" refers to a crystal formed by the combination of an active pharmaceutical ingredient (API) and an eutectic crystal former (CCF) under the action of hydrogen bonds or other non-covalent bonds. The pure state of API and CCF are both solid at room temperature, and there is a fixed stoichiometric ratio between various components. The eutectic crystal is a multi-component crystal, which includes both a binary eutectic crystal formed between two neutral solids and a multi-element eutectic crystal formed between a neutral solid and a salt or solvate.

The term "animal" refers to an animal including mammals, such as humans, companion animals, zoo animals and domestic animals, preferably humans, horses or dogs.

The term "stereoisomer" refers to an isomer produced as a result of different spatial arrangement of atoms in molecules, including cis-trans isomers, enantiomers and conformational isomers.

The term "tautomer" refers to a functional group isomer produced by the rapid movement of an atom in two positions in a molecule, such as keto-enol isomerization and amide-imino alcohol isomerization.

The term "$IC_{50}$" refers to the concentration of a drug or inhibitor required to inhibit half of a given biological process (or a component of the process such as an enzyme, a receptor and a cell).

Unless otherwise specified, the term "content %" of a substance in the pharmaceutical composition of the present application means the weight of the substance as a percentage of the total weight of the pharmaceutical composition.

The term "content %" in the determination of the content of a formulation is the weight of the active drug of the formulation as a percentage of the weight of the active drug feed in the formulation as measured by the determination method.

## Detailed Description of Embodiments

The technical solutions of the present invention will be described in detail by the following examples, but the scope of protection of the present invention includes but is not limited thereto.

The compounds used in the reactions described herein are prepared according to organic synthesis techniques known to those skilled in the art, starting from commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

The structures of the compounds are determined by nuclear magnetic resonance (NMR) or (and) mass spectrometry (MS). The NMR shift ($\delta$) is given in the unit of $10^{-6}$ (ppm). NMR is measured with NMR instruments (Bruker Avance III 400 and Bruker Avance 300); the solvent for determination is deuterated dimethyl sulfoxide (DMSO-$d_6$),

deuterated chloroform ($CDCl_3$) and deuterated methanol ($CD_3OD$); and the internal standard is tetramethylsilane (TMS).

MS is determined with Agilent 6120B (ESI) and Agilent 6120B (APCI).

HPLC is determined with Agilent 1260DAD high pressure liquid chromatograph (Zorbax SB-C18 100 × 4.6 mm, 3.5 μM).

Yantai Huanghai $HSGF_{254}$ or Qingdao $GF_{254}$ silica gel plate is used as a thin layer chromatography silica plate; and the silica gel plate for the thin layer chromatography (TLC) is of the specification of 0.15 mm-0.20 mm, and the specification when separating and purifying a product by thin layer chromatography is 0.4 mm-0.5 mm.

For the column chromatography, Yantai Huanghai silica gel of 200-300 mesh silica gel is generally used as a carrier;

Boc: tert-butoxycarbonyl;
Ts: p-toluenesulfonyl;
Cbz: benzyloxycarbonyl;
TMS: trimethylsilane.

**Example 1: preparation of compound 1**

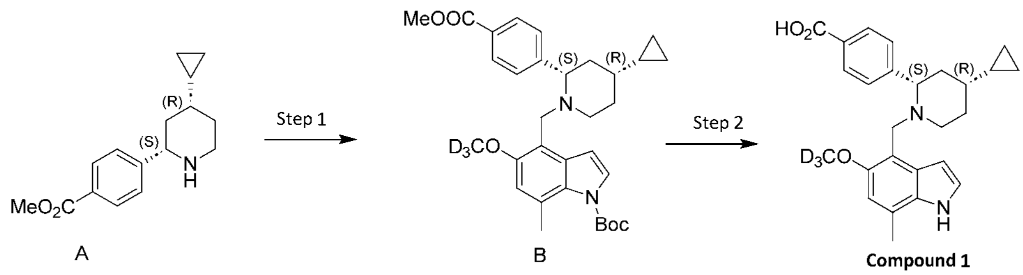


Step 1:

Compound A (162 mg, 0.625 mmol) was dissolved in 5 mL of isopropyl acetate, maleic acid (73 mg, 0.628 mmol) was added, and the mixture was reacted at room temperature for 1 h. The reaction system was concentrated under reduced pressure to yield a crude product (235 mg), the crude product (235 mg) was then dissolved in 10 mL of ethanol, tert-butyl 4-formyl-5-trideuteriomethoxy-7-methyl-1H-indole-1-carboxylate (165 mg, 0.564 mmol) and 20 mg of $Ir(CO)_2$acac were added, and nitrogen replacement was performed three times, and the mixture was heated to 80°C, and reacted under the atmosphere of hydrogen balloon for 16 h. The reaction solution was cooled to room temperature and concentrated under reduced pressure. The resulting crude product was separated and purified by silica gel column chromatography (petroleum ether/ethyl acetate (v/v) = 10 : 1) to afford compound B (200 mg, yield: 60%).

LCMS m/z = 536.3 $[M+1]^+$

Step 2:

Compound B (200 mg, 0.37 mmol) was dissolved in 5 mL of methanol, solid potassium carbonate (257 mg, 1.86 mmol) was added, and the mixture was heated to 80°C and reacted under reflux for 3 h. The reaction solution was cooled to room temperature and concentrated under reduced pressure to afford a crude product. The above-mentioned crude product was dissolved in a mixed solvent of 5 mL of THF and 1 mL of water, and lithium hydroxide monohydrate (155 mg, 3.7 mmol) was added and the mixture was stirred at room temperature for 16 h. The reaction system was concentrated under reduced pressure, and the crude product was subjected to Pre-HPLC (instrument and preparative column: the Glison GX-281 preparative liquid phase chromatographic instrument being used, preparative column model: Sunfire C18, 5 μm, inner diameter × length = 30 mm × 150 mm). Preparation method: the crude product was dissolved in methanol and dimethyl sulfoxide, and filtered with a 0.45 μm filter membrane, so as to prepare a sample liquid. Mobile phase system: acetonitrile/water (containing 0.1% TFA). Gradient elution method: gradient elution with acetonitrile from 5% to 60% (elution time: 15 min). Lyophilization was performed to afford trifluoroacetate of **compound 1** (125 mg).

$^1$H NMR (400 MHz, $CD_3OD$) δ 8.28 - 8.18 (m, 2H), 7.77 - 7.68 (m, 2H), 7.36 - 7.30 (m, 1H), 6.77 (s, 1H), 6.32 (d, 1H), 4.48 (dd, 1H), 4.40 - 4.29 (m, 1H), 4.18 - 4.08 (m, 1H), 3.62 - 3.52 (m, 1H), 3.30 - 3.21 (m, 1H), 2.51 (s, 3H), 2.25 - 2.12 (m, 1H), 2.09 - 1.85 (m, 2H), 1.79 - 1.60 (m, 1H), 1.20 - 1.03 (m, 1H), 0.67 - 0.53 (m, 1H), 0.52 - 0.40 (m, 2H), 0.26 - 0.12 (m, 2H).

LCMS m/z = 422.2 $[M+1]^+$

To 125 mg of the trifluoroacetate of **compound 1** were added 10 mL of 2-methyltetrahydrofuran and 50 mL of saturated sodium bicarbonate solution, the mixture was stirred for 15 min and then extracted, and the organic phase was concentrated to dryness, so as to afford **compound 1** (60 mg).

**Example 1-1: succinate of compound 1**

About 250 mg of **compound 1** was weighed, 7.5 mL of ethanol and 2.5 mL of water were added, and the mixture was stirred at 25°C; 15 mL of an aqueous succinic acid solution with a concentration of 47 mg/mL was added, and the resulting mixture was stirred for 2 h and then subjected to suction filtration, and the filter cake was rinsed with 30% aqueous ethanol and dried under vacuum to afford succinate of **compound 1.**

$^1H$ NMR showed that the ratio of the number of H in the methyl peak (2.42 ppm, 3H) of compound 1 at the chemical shift to the number of H in the methylene peak (2.41 ppm, 4H) of succinic acid was 1 : 1. Therefore, the molar ratio of compound 1 to succinic acid was initially determined to be 1 : 0.75.

**Example 2:**

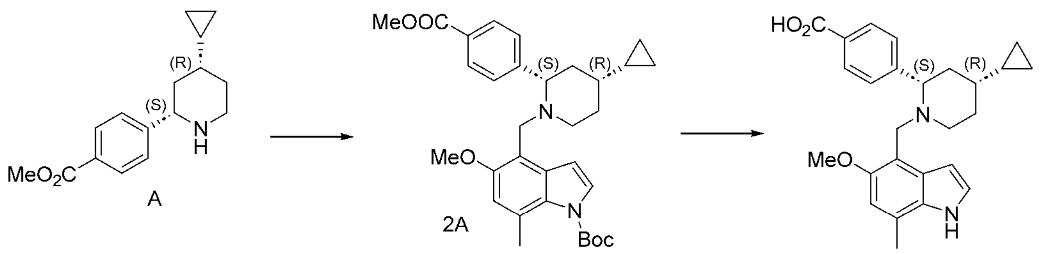

A

2A

Compound 2

With reference to the preparation method for compound 1, compound 2 or trifluoroacetate thereof was obtained.

$^1H$ NMR (400 MHz, $CD_3OD$) of trifluoroacetate of compound 2: δ 8.23 (d, 2H), 7.73 (d, 2H), 7.33 (d, 1H), 6.77 (s, 1H), 6.33 (d, 1H), 4.48 (dd, 1H), 4.38 - 4.31 (m, 1H), 4.17 - 4.09 (m, 1H), 3.76 (s, 3H), 3.62 - 3.53 (m, 1H), 3.30 - 3.21 (m, 1H), 2.51 (s, 3H), 2.25 - 2.11 (m, 1H), 2.05 - 1.88 (m, 2H), 1.78 - 1.60 (m, 1H), 1.21 - 1.05 (m, 1H), 0.65 - 0.53 (m, 1H), 0.51 - 0.39 (m, 2H), 0.24 - 0.14 (m, 2H).

LCMS m/z = 419.2 $[M+1]^+$

**Example 2-1:**

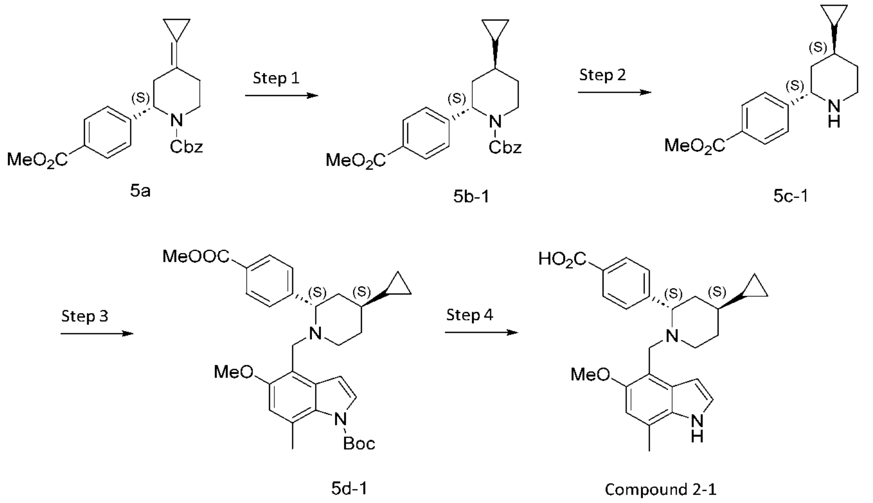


5a

5b-1

5c-1

5d-1

Compound 2-1

Step 1: preparation of 5b-1

5a (3.7 g, 9.45 mmol) was added to 50 mL of ultra-dry DMF, the mixture was placed in a nitrogen atmosphere, solid benzenesulfonyl hydrazide (8.2 g, 47.6 mmol) was added, and the resulting mixture was heated to 100°C and reacted for 16 h. The reaction solution was cooled to room temperature, 100 mL of ethyl acetate was added, and the organic phase was washed three times with 100 mL of saturated aqueous sodium chloride solution, dried over anhydrous sodium sulfate, and concentrated under reduced pressure. The obtained crude product was separated and purified by silica gel column chromatography (petroleum ether/ethyl acetate (v/v) = 15 : 1) to afford 5b-1 (0.7 g, yield: 19%).

Rf value of compound 5b-1: 0.36 (developing agent: ethyl acetate/petroleum ether (v/v) = 1 : 10)

Nuclear magnetic resonances of compound 5b-1:

$^1$H NMR (400 MHz, $CDCl_3$) δ 8.04 - 7.90 (m, 2H), 7.50 - 7.10 (m, 7H), 5.75 - 5.45 (m, 1H), 5.19 (s, 2H), 4.40 - 4.05 (m, 1H), 3.85 (s, 3H), 2.84 - 2.66 (m, 1H), 2.54 - 2.33 (m, 1H), 1.84 - 1.55 (m, 2H), 1.45 - 1.18 (m, 1H), 0.72 - 0.27 (m, 4H), 0.09 - -0.06 (m, 2H).

LCMS m/z = 394.2 $[M+1]^+$

Step 2: preparation of maleate of 5c-1

5b-1 (1.0 g, 2.54 mmol) was dissolved in 10 mL of acetonitrile, and trimethylsilyl iodide (2.54 g, 12.7 mmol) was slowly added in a dropwise manner, and the mixture was stirred at room temperature for 30 min. 10 mL of methanol was added to the reaction system, the pH of the system was adjusted to 2-3 with 2 mol/L dilute hydrochloric acid, the organic phase was extracted with 20 mL of ethyl acetate, and the pH of the aqueous phase was adjusted to 10 with a 2 mol/L sodium hydroxide solution. The mixture was extracted three times by adding 100 mL of dichloromethane, dried over anhydrous sodium sulfate, and concentrated under reduced pressure to afford compound 5c-1 (diastereomer 2) (0.6 g). Compound 5c-1 (diastereomer 2) (0.6 g, 2.31 mmol) was dissolved in 10 mL of isopropyl acetate, maleic acid (270 mg, 2.33 mmol) was added, and the mixture was reacted at room temperature for 16 h. The reaction system was concentrated under reduced pressure to afford maleate (0.85 g) of crude product 5c-1.

Step 3: preparation of 5d-1

The maleate (0.85 g) of 5c-1 was dissolved in 10 mL of ethanol, tert-butyl 4-formyl-5-methoxy-7-methyl-1H-indole-1-carboxylate (0.67 g, 2.32 mmol) (see WO 2015009616 for the synthesis method) and 80 mg of $Ir(CO)_2$acac were added. The mixture was heated to 80°C, and reacted under the atmosphere of hydrogen balloon for 16 h. The reaction solution was cooled to room temperature and concentrated under reduced pressure. The resulting crude product was separated and purified by silica gel column chromatography (petroleum ether/ethyl acetate (v/v) = 15 : 1) to afford 5d-1 (850 mg, yield: 69%).

Nuclear magnetic resonances of compound 5d-1:

$^1$H NMR (400 MHz, $CDCl_3$) δ 8.08 - 7.96 (m, 2H), 7.67 - 7.56 (m, 2H), 7.48 (d, 1H), 6.71 (d, 1H), 6.67 (s, 1H), 3.91 (s, 3H), 3.80 (s, 3H), 3.74 - 3.64 (m, 1H), 3.60 - 3.50 (m, 1H), 3.44 - 3.33 (m, 1H), 2.76 - 2.65 (m, 1H), 2.59 (s, 3H), 2.45 - 2.32 (m, 1H), 1.96 - 1.50 (m, 13H), 1.23 - 1.10 (m, 1H), 0.90 - 0.75 (m, 1H), 0.57 - 0.41 (m, 2H), 0.10 - 0.01 (m, 2H).

LCMS m/z = 533.3 $[M+1]^+$

Step 4: preparation of trifluoroacetate of compound 2-1

5d-1 (850 mg, 1.596 mmol) was dissolved in 10 mL of methanol, solid potassium carbonate (1.1 g, 7.96 mmol) was added, and the mixture was heated to 80°C and reacted under reflux for 3 h. The reaction solution was cooled to room temperature and concentrated under reduced pressure to afford a crude product. The above-mentioned crude product was dissolved in a mixed solvent of 10 mL of THF and 2 mL of water, and lithium hydroxide monohydrate (670 mg, 15.97 mmol) was added and the mixture was stirred at room temperature for 16 h. The reaction system was concentrated under reduced pressure, and the crude product was subjected to Pre-HPLC (instrument and preparative column: the Glison GX-281 preparative liquid phase chromatographic instrument being used, preparative column model: Sunfire C18, 5 μm, inner diameter × length = 30 mm × 150 mm). Preparation method: the crude product was dissolved in methanol and dimethyl sulfoxide, and filtered with a 0.45 μm filter membrane, so as to prepare a sample liquid. Mobile phase system: acetonitrile/water (containing 0.1% TFA). Gradient elution method: gradient elution with acetonitrile from 5% to 60% (elution time: 15 min). Lyophilization was performed to afford trifluoroacetate (400 mg) of compound 2-1.

$^1H$ NMR (400 MHz, $CD_3OD$) δ 8.29 - 8.18 (m, 2H), 7.79 - 7.72 (m, 2H), 7.37 - 7.32 (m, 1H), 6.78 (s, 1H), 6.38 (d, 1H), 4.83 - 4.78 (m, 1H), 4.41 - 4.32 (m, 1H), 4.30 - 4.20 (m, 1H), 3.77 (s, 3H), 3.65 - 3.42 (m, 2H), 2.51 (s, 3H), 2.40 - 2.25 (m, 1H), 2.18 - 2.02 (m, 2H), 1.98 - 1.82 (m, 1H), 1.52 - 1.38 (m, 1H), 1.18 - 1.04 (m, 1H), 0.72 - 0.60 (m, 2H), 0.25 - 0.17 (m, 2H).

LCMS m/z = 419.2 $[M+1]^+$

The trifluoroacetate of compound 5-1 had obvious $^1H$-$^1H$ NOESY signals on the C1 hydrogen of the piperidine ring and the C6 hydrogen of the cyclopropyl, which proved that the configuration of compound 5-1 was as shown in the following formula:

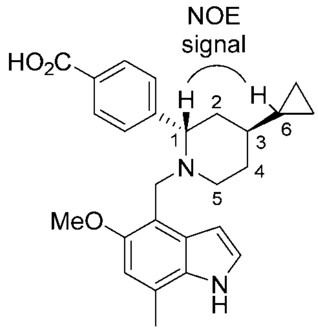


Example 3:

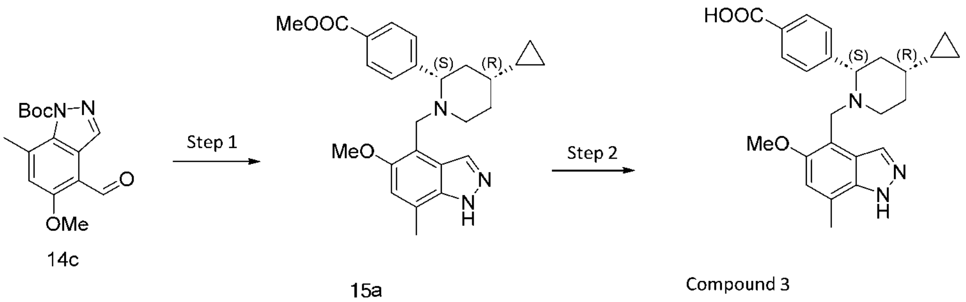


Step 1:

14c (0.40 g, 1.38 mmol), maleate (0.62 g) of compound A and a 4Å molecular sieve (100 mg) were added to a reaction flask, glacial acetic acid (0.5 mL) and dichloroethane (10 mL) were added, and the mixture was heated to 60°C and reacted for 16 h. The reaction system was cooled to room temperature, and sodium triacetoxyborohydride (0.88 g, 4.15 mmol) was added. The resulting mixture was reacted at room temperature for 6 h. 20 mL of saturated aqueous sodium bicarbonate solution was added in dropwise manner to the reaction system, the insoluble matter was filtered by means of diatomaceous earth, and the filtrate was collected, extracted with dichloromethane (20 mL × 3), dried over anhydrous sodium sulfate, and concentrated under reduced pressure. The crude product was separated and purified with a silica gel chromatography column (ethyl acetate/petroleum ether (v/v) = 10 : 1 to 1 : 0) to afford crude product 15a (102 mg).

LCMS m/z = 434.3 $[M+1]^+$

Step 2:

To the above-mentioned crude product 15a (102 mg) were added solid sodium hydroxide (100 mg, 2.50 mmol), 5 mL of methanol and 5 mL of water, and the mixture was heated to 75°C and reacted under reflux for 0.5 h. The reaction solution was cooled to room temperature and concentrated under reduced pressure, and the crude product was subjected to Pre-HPLC (instrument and preparative column: SHIMADZU LC-20AP preparative liquid phase chromatographic instrument being used, preparative column model: Phenomenex C18). Preparation method: the crude product was dissolved in methanol and dimethyl sulfoxide, and filtered with a 0.45 μm filter membrane, so as to prepare a sample liquid. Mobile phase system: acetonitrile/aqueous solution containing 10 mmol/L ammonium bicarbonate. Gradient elution method: gradient elution with acetonitrile from 15% to 45% (elution time: 10 min). Lyophilization was performed to obtain compound 3 (9 mg, two-step yield from compound 14c: 2%).

$^1$H NMR (400 MHz, $CD_3OD$) δ 8.16 - 8.06 (m, 2H), 7.98 (s, 1H), 7.65 - 7.56 (m, 2H), 7.06 (s, 1H), 4.30 - 3.83 (m, 3H), 3.79 (s, 3H), 3.40 - 3.32 (m, 1H), 2.56 (s, 3H), 2.17 - 2.03 (m, 1H), 1.99 - 1.76 (m, 2H), 1.74 - 1.52 (m, 1H), 1.09 - 0.93 (m, 1H), 0.67 - 0.52 (m, 1H), 0.50 - 0.36 (m, 2H), 0.23 - 0.10 (m, 2H).

LCMS m/z = 420.2 $[M+1]^+$

**Example 4:**

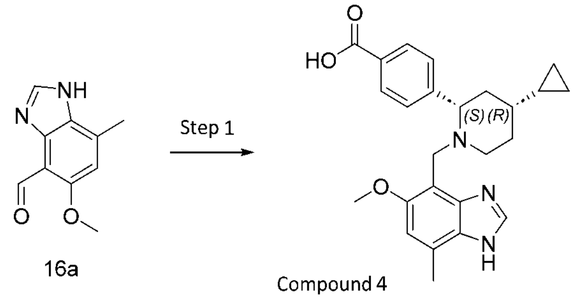


16a

Compound 4

16a (see WO 2022028507 for synthesis method) (165 mg, 0.868 mmol) was dissolved in 10 mL of dichloromethane, triethylamine (260.78 mg, 2.6 mmol), $(Boc)_2O$ (227.0 mg, 1.04 mmol) and 4-dimethylaminopyridine (5.3 mg, 0.043 mmol) were added in sequence, and the mixture was reacted at room temperature for 1 h. 50 mL of dichloromethane was added to the reaction solution, and the organic phase was washed with 0.5 mol/L aqueous hydrochloric acid solution (30 mL ×2), water (30 mL × 2) and saturated sodium chloride solution (30 mL × 2) in sequence, dried over anhydrous sodium sulfate, and concentrated under reduced pressure. The crude product was separated and purified with silica gel chromatography column (petroleum ether/ethyl acetate (v/v) = 3 : 1) to afford crude product 1 (0.22 g). The above-mentioned crude product 1 (70 mg) was dissolved in 10 mL of 1,2-dichloroethane, the maleate (91 mg) of compound A, solid sodium bicarbonate (61 mg, 0.726 mmol) and 0.02 mL of acetic acid were added in sequence, and the mixture was stirred at room temperature for 0.5 h, then heated to 70°C and reacted for 16 h. The reaction solution was cooled to room temperature, and sodium triacetoxyborohydride (102 mg, 0.48 mmol) was added. The resulting mixture was reacted at room temperature for 16 h. 50 mL of saturated aqueous sodium bicarbonate solution was added to the reaction solution, the mixture was extracted with ethyl acetate (50 mL × 2), and the organic phase was washed with saturated sodium chloride solution (30 mL × 2), dried over anhydrous sodium sulfate, and concentrated under reduced pressure. The crude product was separated and purified with silica gel chromatography column (petroleum ether/ethyl acetate (v/v) = 1 : 3) to afford a crude product (40 mg), which was subjected to Pre-HPLC (instrument and preparative column: SHIMADZU LC-20AP preparative liquid phase chromatographic instrument being used, preparative column model: Phenomenex C18). Preparation method: the crude product was dissolved in methanol and dimethyl sulfoxide, and filtered with a 0.45 μm filter membrane, so as to prepare a sample liquid. Mobile phase system: acetonitrile/aqueous solution containing 10 mmol/L ammonium bicarbonate. Gradient elution method: gradient elution with acetonitrile from 35% to 65% (elution time: 10 min), affording crude product 2 (8 mg). The above-mentioned crude product 2 (8 mg) was dissolved in a mixed solvent of 2 mL of methanol and 2 mL of water, then solid sodium hydroxide (4 mg, 0.1 mmol) was added, and the mixture was heated to 75°C and reacted for 2 h. The reaction solution was cooled to room temperature, the pH was adjusted to 8 with 1 mol/L aqueous hydrochloric acid solution, and the crude product was subjected to Pre-HPLC (instrument and preparative column: SHIMADZU LC-20AP preparative liquid phase chromatographic instrument being used, preparative column model: Phenomenex C18). Preparation method: the crude product was dissolved in methanol and dimethyl sulfoxide, and filtered with a 0.45 μm filter membrane, so as to prepare a sample liquid. Mobile phase system: acetonitrile/aqueous solution containing 10 mmol/L ammonium bicarbonate. Gradient elution method: gradient elution with acetonitrile from 35% to 65% (elution time: 10 min), affording compound 4 (4 mg, yield: 3%).

$^1$H NMR (400 MHz, $CD_3OD$) δ 8.00 (s, 1H), 7.94 - 7.84 (m, 2H), 7.48 - 7.36 (m, 2H), 6.68 (s, 1H), 4.20 - 3.70 (m, 3H), 3.59 (s, 3H), 3.23 - 3.09 (m, 1H), 2.90 - 2.66 (m, 1H), 2.38 (s, 3H), 2.00 - 1.87 (m, 1H), 1.81 - 1.66 (m, 2H), 1.60 - 1.40 (m, 1H), 0.96 - 0.78 (m, 1H), 0.50 - 0.35 (m, 1H), 0.34 - 0.20 (m, 2H), 0.07 - 0.05 (m, 2H).

LCMS m/z = 420.3 $[M+1]^+$

**Formulation examples:**

Unless otherwise specified, a proportion (%) referred to the weight of a component as a percentage of the total weight of the prescription.

Unless the succinate of compound 1 was otherwise specified, the molar ratio thereof was 1 : 0.75.

1. Formulation 1-1: Specification 200 mg/tablet (calculated based on a free base):

Table 2 Prescription of formulation (300 tablets prepared)

| Name of raw/auxiliary material | Amount (mg/tablet) | Proportion (%) |
|---|---|---|
| Succinate of compound 1 | 248 | 39.81 |
| Copovidone | 20 | 3.21 |
| Silica | 10 | 1.61 |
| Croscarmellose sodium | 20 | 3.21 |
| Microcrystalline cellulose | 100 | 16.05 |
| Lactose | 220 | 35.31 |
| Magnesium stearate | 5 | 0.80 |
| Total | 623 | 100 |

2. Formulation 1-2: Specification 200 mg/tablet (calculated based on a free base):

Table 3 Prescription of formulation (300 tablets prepared)

| Name of raw/auxiliary material | Amount (mg/tablet) | Proportion (%) |
|---|---|---|
| Succinate of compound 1 | 248 | 39.81 |
| Hypromellose | 25 | 4.01 |
| Silica | 10 | 1.61 |
| Crospovidone | 20 | 3.21 |
| Microcrystalline cellulose | 215 | 34.51 |
| Lactose | 100 | 16.05 |
| Magnesium stearate | 5 | 0.80 |
| Total | 623 | 100 |

3. Formulation 1-3: Specification 200 mg/tablet (calculated based on a free base): (300 tablets prepared)

Table 4 Prescription of formulation

| Name of raw/auxiliary material | Amount (mg/tablet) | Proportion (%) |
|---|---|---|
| Succinate of compound 1 | 248 | 39.81 |
| Hydroxypropyl cellulose | 25 | 4.01 |
| Silica | 10 | 1.61 |
| Crospovidone | 20 | 3.21 |
| Microcrystalline cellulose | 210 | 33.71 |
| Mannitol | 100 | 16.05 |
| Sodium stearyl fumarate | 10 | 1.61 |

(continued)

| Name of raw/auxiliary material | Amount (mg/tablet) | Proportion (%) |
|---|---|---|
| Total | 623 | 100 |

Prescription formulations 1-1 to 1-3 described above were prepared by using a process comprising the following steps:

1) weighing, involving: weighing each raw/auxiliary material according to the prescription (converted content of active pharmaceutical ingredient);
2) mixing, involving: mixing an active drug, a binder, a glidant, a disintegrant and a filler for 5 min, adding a lubricant and mixing same for 2 min; and
3) tableting, involving: tableting the powder obtained in 2) using a shallow concave die with a diameter of 11 mm, with the tablet weight controlled at 575 mg and the hardness controlled at 70 N-130 N.

4. Formulation 1-4: Specification 200 mg/tablet (calculated based on a free base):

Table 5 Prescription of formulation (300 tablets prepared)

| Name of raw/auxiliary material | Amount (mg/tablet) | Proportion (%) |
|---|---|---|
| Compound 1 | 200 | 34.8 |
| Microcrystalline cellulose | 325 | 56.5 |
| Povidone | 25 | 4.3 |
| Crospovidone | 20 | 3.5 |
| Magnesium stearate | 5 | 0.9 |
| Total | 575 | 100 |

A prescription process for formulation 1-4 comprised:

premixing, involving: mixing an active ingredient with microcrystalline cellulose for 1 min, then screening same by using a crushing granulator, placing the screened mixture and crospovidone into a wet granulator, and mixing same for 5 min, so as to obtain a mixture;
binder preparation, involving: preparing 8.7% povidone solution;
granulation, involving: running the wet granulator, slowly adding 8.0% povidone solution and stirring same for about 120 s, so as to prepare a soft material;
whole granule drying, involving: wetting the soft material with a 14-mesh screen using a rocking granulator, so as to obtain wet granules, drying the wet granules in an oven at 80°C, with the moisture content controlled to be 3.5% or less, and drying the whole granules by passing through an 18-mesh screen using the rocking granulator, so as to obtain granulated dry granules for later use;
total mixing, involving: placing the granulated dry granules into a hopper mixer, adding magnesium stearate, and mixing same for 8 min, so as to obtain a total blend of granules; and
tableting, involving: according to the theoretical tablet weight, performing tabletting by using a shallow concave die with a diameter of 10 mm, with the tablet weight controlled to be within ± 5% of the theoretical tablet weight and the hardness controlled at 70 N-130 N, so as to obtain a plain tablet.

5. Formulation 1-5: Specification 50 mg/tablet (calculated based on a free base)

Table 6 Prescription of formulation (300 tablets prepared)

| Name of raw/auxiliary material | Amount (mg/tablet) | Proportion (%) |
|---|---|---|
| Succinate of compound 1 | 62 | 23.66 |

(continued)

| Name of raw/auxiliary material | Amount (mg/tablet) | Proportion (%) |
|---|---|---|
| Povidone K30 | 10 | 3.82 |
| Croscarmellose sodium | 10 | 3.82 |
| Lactose | 179 | 68.32 |
| Magnesium stearate | 1 | 0.38 |
| Total | 262 | 100 |

6. Formulation 1-6: Specification 100 mg/tablet (calculated based on a free base)

Table 7 Prescription of formulation (300 tablets prepared)

| Name of raw/auxiliary material | Amount (mg/tablet) | Proportion (%) |
|---|---|---|
| Succinate of compound 1 | 124 | 29.25 |
| Povidone K30 | 40 | 9.43 |
| Crospovidone | 20 | 4.72 |
| Microcrystalline cellulose | 236 | 55.66 |
| Magnesium stearate | 4 | 0.94 |
| Total | 424 | 100 |

7. Formulation 1-7: Specification 200 mg/tablet (calculated based on a free base)

Table 8 Prescription of formulation (300 tablets prepared)

| Name of raw/auxiliary | Amount | Proportion (%) |
|---|---|---|
| material | (mg/tablet) | |
| Succinate of compound 1 | 248 | 33.16 |
| Povidone K90 | 20 | 2.67 |
| Sodium carboxymethyl starch | 40 | 5.35 |
| Microcrystalline cellulose | 330 | 44.12 |
| Starch | 100 | 13.37 |
| Magnesium stearate | 10 | 1.34 |
| Total | 748 | 100 |

8. Formulation 1-8: Specification 200 mg/tablet (calculated based on a free base)

Table 9 Prescription of formulation (300 tablets prepared)

| Name of raw/auxiliary material | Amount (mg/tablet) | Proportion (%) |
|---|---|---|
| Succinate of compound 1 | 62 | 31.47 |
| Hypromellose | 15 | 7.61 |
| Sodium carboxymethyl starch | 15 | 7.61 |

(continued)

| Name of raw/auxiliary material | Amount (mg/tablet) | Proportion (%) |
|---|---|---|
| Microcrystalline cellulose | 50 | 25.38 |
| Starch | 50 | 25.38 |
| Magnesium stearate | 5 | 2.54 |
| Total | 197 | 100 |

9. Formulation 1-9: Specification 400 mg/tablet (calculated based on a free base)

Table 10 Prescription of formulation (300 tablets prepared)

| Name of raw/auxiliary material | Amount (mg/tablet) | Proportion (%) |
|---|---|---|
| Succinate of compound 1 | 496 | 62.31 |
| Hypromellose | 40 | 5.03 |
| Sodium carboxymethyl starch | 50 | 6.28 |
| Microcrystalline cellulose | 150 | 18.84 |
| Lactose | 50 | 6.28 |
| Magnesium stearate | 10 | 1.26 |
| Total | 796 | 100 |

10. Formulation 1-10: Specification 30 mg/tablet (calculated based on a free base)

Table 11 Prescription of formulation (300 tablets prepared)

| Name of raw/auxiliary material | Amount (mg/tablet) | Proportion (%) |
|---|---|---|
| Succinate of compound 1 | 37.2 | 8.30 |
| Hypromellose | 30 | 6.70 |
| Sodium carboxymethyl starch | 25 | 5.58 |
| Microcrystalline cellulose | 250 | 55.80 |
| Lactose | 100 | 22.32 |
| Magnesium stearate | 5.8 | 1.29 |
| Total | 440 | 100 |

11. Formulation 1-11: Specification 500 mg/tablet (calculated based on a free base)

Table 12 Prescription of formulation (300 tablets prepared)

| Name of raw/auxiliary material | Amount (mg/tablet) | Proportion (%) |
|---|---|---|
| Succinate of compound 1 | 620 | 63.27 |
| Hypromellose | 50 | 5.10 |
| Sodium carboxymethyl starch | 50 | 5.10 |
| Microcrystalline cellulose | 100 | 10.20 |

(continued)

| Name of raw/auxiliary material | Amount (mg/tablet) | Proportion (%) |
|---|---|---|
| Lactose | 150 | 15.31 |
| Magnesium stearate | 10 | 1.02 |
| Total | 980 | 100 |

12. Formulation 1-12: Specification 700 mg/tablet (calculated based on a free base)

Table 13 Prescription of formulation (300 tablets prepared)

| Name of raw/auxiliary material | Amount (mg/tablet) | Proportion (%) |
|---|---|---|
| Compound 1 | 700 | 66.0 |
| Hydroxypropyl cellulose | 40 | 3.8 |
| Sodium carboxymethyl starch | 60 | 5.7 |
| Microcrystalline cellulose | 100 | 9.4 |
| Lactose | 150 | 14.2 |
| Magnesium stearate | 10 | 0.9 |
| Total | 1060 | 100 |

13. Formulation 1-13: Specification 800 mg/tablet (calculated based on a free base)

Table 14 Prescription of formulation (300 tablets prepared)

| Name of raw/auxiliary material | Amount (mg/tablet) | Proportion (%) |
|---|---|---|
| Succinate of compound 1 | 992 | 70.86 |
| Hypromellose | 50 | 3.57 |
| Sodium carboxymethyl starch | 58 | 4.14 |
| Microcrystalline cellulose | 120 | 8.57 |
| Lactose | 170 | 12.14 |
| Magnesium stearate | 10 | 0.71 |
| Total | 1400 | 100 |

Prescription formulations 1-5, 1-6, 1-7, 1-8, 1-9, 1-10, 1-11, 1-12 and 1-13 were prepared using a prescription process similar to that for prescription formulation 1-4. Purified water was used as a wetting agent during the preparation of a formulation.

**Formulation test examples:**

**1. Content determination**

**High performance liquid chromatography** (General Chapters in Volume IV of the Chinese Pharmacopoeia 2020 Edition) test: filler: octadecylsilane chemically bonded silica (ACE Excel 5 C18 PFP, 4.6 mm x 250 mm, 5 μm; ); mobile phase A: 0.02 mol/L ammonium acetate buffered salt-methanol (850 : 150) (prepared by weighing 1.54 g of ammonium acetate, adding 1000 ml of water to dissolve same, filtering same with a 0.45 μm filter membrane, and mixing 850 ml of the filtrate with 150 ml of methanol until uniform); mobile phase B: acetonitrile; mobile phase A-mobile phase B = 80 : 20, isocratic elution: 30 min; detection wavelength: 222 nm; column temperature: 30°C; and flow rate: 1.0 ml/min.

The contents of examples 1-1 to 1-13 above were determined, and the content test results are shown in Table 15 below.

Table 15 Content determination results

| Example | Content (%) |
|---|---|
| 1-1 | 100.0 |
| 1-2 | 98.2 |
| 1-3 | 97.9 |
| 1-4 | 99.2 |
| 1-5 | 100.3 |
| 1-6 | 101.2 |
| 1-7 | 100.4 |
| 1-8 | 100.9 |
| 1-9 | 100.3 |
| 1-10 | 100.4 |
| 1-11 | 100.9 |
| 1-12 | 100.5 |
| 1-13 | 99.4 |

Conclusion: the contents of the formulations prepared using the technique of the present invention all met the quality requirements.

**2. Dissolution rate determination**

The dissolution rates of examples 1-1 to 1-13 described above were determined.

Test method:

Medium: dissolution medium with a pH of 1.0; volume: 900 mL; method: paddle method; rotating speed: 50 rpm; time: 30 min; and temperature: 37°C ± 0.5°C.

The test results are shown in Table 16 below:

Table 16 Dissolution rate determination

| Example | Dissolution rate (%) |
|---|---|
| 1-1 | 91.0 |
| 1-2 | 87.8 |
| 1-3 | 86.0 |
| 1-4 | 91.7 |
| 1-5 | 94.3 |
| 1-6 | 82.8 |
| 1-7 | 84.8 |
| 1-8 | 90.8 |
| 1-9 | 81.6 |
| 1-10 | 91.3 |
| 1-11 | 81.6 |
| 1-12 | 82.6 |

(continued)

| Example | Dissolution rate (%) |
|---|---|
| 1-13 | 93.9 |

Conclusion: the formulations prepared using the technique of the present invention had high dissolution rates.

**Biological test examples**

**1. Test of inhibitory effect on the complement alternative pathway**

The inhibitory effect of the compounds on the complement alternative pathway was detected using the IBL alternative pathway kit (COMPL AP330 RUO). Serum from healthy volunteers was collected and 1 portion was diluted with a buffer of 20 mM EDTA in gelatin (0.15 mM $CaCl_2$, 141 mM NaCl, 4.5 mM $MgCl_2$, 4.2 mM HEPES, and 0.1% gelatin at pH 7.4) to 50% (v/v), and 1 portion was diluted to 50% (v/v) with a buffer of 20 mM EGTA in gelatin. The compounds were prepared in DMSO to a working solution 100 times the final concentration. Before use, 50% of the above serum was diluted 1/18 with an AP diluent in the kit. 1 part of the compound working solution (v/v) or DMSO (positive control PC) was added to 99 parts of the diluted serum containing EGTA for pretreatment for 30 min, and 1 part of DMSO (v/v) was added to 99 parts of the diluted serum containing EDTA, as negative control (NC). The serum pretreated with the above compounds (or DMSO) was added at 100 mL/well to an LPS-coated 96-well plate and incubated at 37°C for 1 h, and the plate was washed 3 times with washing solution in the kit, and the conjugated chromogenic substrate was added according to the instruction in the kit, and the absorbance was read at 405 nm with a microplate reader. The inhibitory activity of the compounds on alternative pathway was calculated using the following formula.

$$\text{Inhibition rate}\ \% = 100\% \times \frac{OD^{\mathrm{CPD}} - OD^{\mathrm{NC}}}{OD^{\mathrm{PC}} - OD^{\mathrm{NC}}}$$

$OD^{CPD}$ was the OD value when the inhibitor was added, $OD^{NC}$ was the OD value of the negative control, and $OD^{PC}$ was the OD value of the positive control.

Graphpad was used to perform nonlinear regression on the final concentrations and inhibition rates of the compounds to obtain the $IC_{50}$ of the compounds for inhibiting the alternative pathway.

The results of the $IC_{50}$ values for inhibiting the alternative pathway are shown in Table 17.

Table 17 $IC_{50}$ values of the compounds of the present invention for inhibiting the alternative pathway

| Serial No. | Compound No. | $IC_{50}$ (nM) |
|---|---|---|
| 1 | Trifluoroacetate of compound 1 | A |
| 2 | Trifluoroacetate of compound 2 | A |
| 3 | Trifluoroacetate of compound 2-1 | A |
| A: < 200 nM; | | |

Conclusion: the compounds of the present invention had significant inhibitory effect on the complement alternative pathway.

**2. Pharmacokinetic test in rats**

**Experimental objective:** in this experiment, a single dose of each test compound was administered to SD rats intravenously and intragastrically, and the concentrations of the test compounds in plasma of rats were measured to evaluate the pharmacokinetic characteristics of the test compounds in rats.

**Experimental animals:** male SD rats, 200-250 g, 6-8 weeks old, 6 rats/compound, were purchased from Chengdu Dossy Experimental Animals Co., Ltd.

**Experimental method:** on the day of the experiment, 6 SD rats were randomly grouped according to their body weights. The rats were fasted with water available for 12-14 h one day before the administration, and were fed 4 h after the administration.

Table 18

| Group | Number Male | Administration information Test compound | Administration dosage * (mg/kg) | Administration concentration (mg/mL ) | Administration volume (mL/kg ) | Collected sample | Mode of administration | Vehicle |
|---|---|---|---|---|---|---|---|---|
| G1 | 3 | Compound of the present invention | 10 | 1 | 10 | Plasma | Oral administration (Intragastric administration) | 0.5% MC |
| G2 | 3 | Compound of the present invention | 1 | 0.2 | 5 | Plasma | Intravenous injection | 5% DMA + 5% Soluto 1+ 90% Saline |
| * Dosage is calculated based on free base. | | | | | | | | |

**Sampling:** before and after the administration, 0.15 mL of blood was taken from the orbits under isoflurane anesthesia and placed in an EDTAK2 centrifuge tube. Centrifugation was performed at 5000 rpm at 4°C for 10 min, and the plasma was collected.

The plasma collection time points for IV&PO groups were 0, 5 min, 15 min, 30 min, 1 h, 2 h, 4 h, 6 h, 8 h, and 24 h.

Before analysis and detection, all samples were stored at -60°C. The samples were analyzed quantitatively by LC-MS/MS.

Table 19 Pharmacokinetic parameters of compounds of the present invention in plasma of rats Test compound

| Test compound | Mode of administration * | Cmax (ng.mL-1) | $T_{1/2}$ (h) | Tmax (h) | $AUC_{0-24\ h}$ (ng/mL·h) | F% |
|---|---|---|---|---|---|---|
| Trifluoroacetate of compound 1 | i.g. (10 mg/kg) | 1128 | 3.20 | - | 9164 | 72.1 |
| LNP023 | i.g. (10 mg/kg) | 867 | 3.39 | 3.5 | 9867 | 49 |
| * Note: i.g. (intragastrically) administration of the compounds | | | | | | |

Conclusion: the compounds of the present invention had good oral absorption in rats. Compared with LNP023, the trifluoroacetate of compound 1 had a higher maximum plasma concentration and higher oral bioavailability in rats.

Structure of control compound (LNP023): 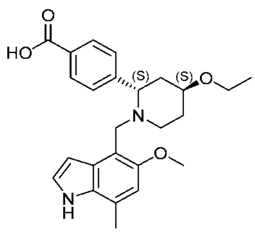 .

**3. Pharmacokinetic test in dogs**

**Experimental objective:** in this experiment, a single dose of test compounds was administered to beagle dogs intravenously and intragastrically, and the concentrations of the test compounds in plasma of beagle dogs were measured to evaluate pharmacokinetic characteristics of the test compounds in beagle dogs.

**Experimental animals:** male beagle dogs, 8-11 kg, 1-3 years old, 6 dogs/compound, were purchased from Beijing Marshall Biotechnology Co., Ltd.

**Experimental method:** on the day of the experiment, 6 beagle dogs were randomly grouped according to their body weights. The beagle dogs were fasted with water available for 14-18 h one day before the administration, and were fed 4 h after the administration.

Table 20

| Gro up | Num ber | Administration information | | | | | | |
|---|---|---|---|---|---|---|---|---|
| | Mal e | Test comp ound | Admi nistrat ion dosag e* (mg/k g) | Admini stration concent ration (mg/m L) | Admi nistrat ion volum e (mL/k g) | Collec ted sampl e | Mode of adminis tration | Vehicle |
| G1 | 3 | Comp ound of the presen t invent ion | 5 | 1 | 5 | Plasm a | Oral adminis tration (Intraga stric adminis tration) | 0.5% MC (containi ng 0.5% Tween 80) |
| G2 | 3 | Comp ound of the presen t invent ion | 1 | 0.5 | 2 | Plasm a | Intrave nous injectio n | 5% DMA + 5% Solutol + 90% Saline |
| * Dosage is calculated based on free base. | | | | | | | | |

**Sampling:** before and after administration, 1.0 mL of blood was taken from the jugular veins or limb veins, and placed in an EDTAK2 centrifuge tube. Centrifugation was performed at 5000 rpm at 4°C for 10 min, and the plasma was collected.

The plasma collection time points for IV&PO groups were 0, 5 min, 15 min, 30 min, 1 h, 2 h, 4 h, 6 h, 8 h, 10 h, 12 h, and 24 h.

Before analysis and detection, all samples were stored at -60°C. The samples were analyzed quantitatively by LC-MS/MS.

Table 21 Pharmacokinetic parameters of compounds of the present invention in plasma of dogs

| Test compound | Mode of administration* | $AUC_{0-t}$ (ng/mL-h) | $T_{1/2}$ (h) | F% |
|---|---|---|---|---|
| Trifluoroacetate of compound 1 | i.g. (5 mg/kg) | 45447 | 15.1 | 75.5 |
| Trifluoroacetate of LNP023 | i.g. (4 mg/kg) | 19129 | 7.06 | 45.9 |
| * Note: i.g. (intragastrically) administration of the compounds | | | | |

Conclusion: the compounds of the present invention had good oral absorption in beagle dogs.

**4. Pharmacokinetic test in monkeys**

**Experimental objective:** in this experiment, a single dose of test compounds was administered to cynomolgus monkeys intravenously and intragastrically, and the concentrations of the test compounds in plasma of monkeys were measured to evaluate pharmacokinetic characteristics of the test compounds in monkeys.

**Experimental animals:** male cynomolgus monkeys, 3-5 kg, 3-6 years old, 6 monkeys/compound, were purchased from Suzhou Xishan Biotechnology Co., Ltd.

**Experimental method:** on the day of the experiment, 6 monkeys were randomly grouped according to their body weights. The monkeys were fasted with water available for 14-18 h one day before the administration, and were fed 4 h after the administration.

Table 22

| Group | Number | Administration information | | | | | | |
|---|---|---|---|---|---|---|---|---|
| | Male | Test compound | Administration dosage * (mg/kg ) | Administration concentration (mg/mL) | Administration volume (mL/kg ) | Collected sample | Mode of administration | Vehicle |
| G1 | 3 | Compound of the present invention | 5 | 1 | 5 | Plasma | Oral administration (Intragastric administration) | 0.5% MC (containing 0.5% Tween 80) |
| G2 | 3 | Compound of the present | 1 | 0.5 | 2 | Plasma | Intravenous injection | 5% DMA + 5% Solutol + 90% |
| | | invention | | | | | | Saline |
| * Dosage is calculated based on free base. | | | | | | | | |

**Sampling:** before and after administration, 1.0 mL of blood was taken from the limb veins, and placed in an EDTAK2 centrifuge tube. Centrifugation was performed at 5000 rpm at 4°C for 10 min, and the plasma was collected.

The plasma collection time points for IV&PO groups were 0, 5 min, 15 min, 30 min, 1 h, 2 h, 4 h, 6 h, 8 h, 10 h, 12 h, and 24 h.

Before analysis and detection, all samples were stored at -60°C. The samples were analyzed quantitatively by LC-MS/MS.

Table 23 Pharmacokinetic parameters of compounds of the present invention in plasma of monkeys

| Test compound | Mode of administration* | $AUC_{0-t}$ (ng/mL·h) | F% |
|---|---|---|---|
| Trifluoroacetate of compound 1 | i.g. (5 mg/kg) | 48279 ± 3664 | 98.3 |
| Trifluoroacetate of compound 2 | i.g. (5 mg/kg) | 38759 ± 1429 | 54.1 |
| Trifluoroacetate of LNP023 | i.g. (5 mg/kg) | 13188 ± 1472 | 35.2 |
| * Note: i.g. (intragastrically) administration of the compounds. | | | |

Conclusion: the compounds of the present invention had good oral absorption in monkeys. Compared with the trifluoroacetate of LNP023, the trifluoroacetate of compound 1 had higher oral exposure and bioavailability in monkeys.

**5. Test of Caco2 permeability**

In the experiment, a monolayer of Caco-2 cells was used, and incubated in triplicate in a 96-well Transwell plate. A transport buffer solution (HBSS, 10 mM HEPES, pH 7.4 ± 0.05) containing the compound of the present invention (2 μM) or the control compound digoxin (10 μM), nadolol (2 μM) or metoprolol (2 μM) was added to the administration end hole on the apical side or basal side. DMSO-containing transport buffer solution was added to the corresponding receiving end hole. After incubation for 2 hours at 37°C ± 1°C, the cell plate was removed, and an appropriate amount of samples was taken from the apical side and basal side and transferred to a new 96-well plate. Subsequently, acetonitrile containing an internal standard was added to precipitate the protein. The samples were analyzed using LC MS/MS, and the concentrations of the compounds of the present invention and the control compounds were determined. Concentration data were used to calculate apparent permeability coefficients for transport from the apical side to the basal side, and from the basal side to the apical side of the cell monolayer, and thus to calculate the efflux ratio. The integrity of the cell monolayer after 2 hours of incubation was evaluated by leakage of Lucifer Yellow.

Table 24 Caco2 test results of compound of the present invention

| Test compound | Mean $P_{app}$ ($10^{-6}$ cm/s) | | Efflux Ratio |
|---|---|---|---|
| | A to B | B to A | |
| Trifluoroacetate of compound 1 | 1.77 | 19.4 | 11.0 |
| Trifluoroacetate of LNP023 | 0.570 | 14.4 | 25.3 |

Conclusion: the compound of the present invention had good Caco2 permeability. Compared with the trifluoroacetate of LNP023, the trifluoroacetate of compound 1 had better permeability and a lower efflux ratio.

**Claims**

1. A pharmaceutical composition, comprising an active ingredient A and a pharmaceutically acceptable excipient, **characterized in that** the active ingredient A is selected from a compound represented by general formula (I) or a stereoisomer, a tautomer, a deuterated form, a solvate, a prodrug, a metabolite, a pharmaceutically acceptable salt or an eutectic crystal thereof,

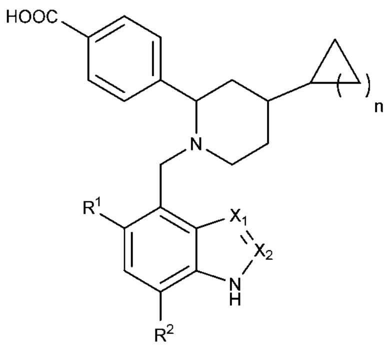
(I)

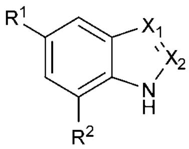

is selected from

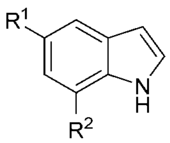
,
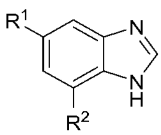
, or
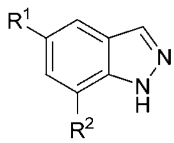
;

$R^1$ is selected from $-OCH_3$ or $-OCD_3$;
$R^2$ is selected from $-CH_3$ or $-CD_3$;
n is selected from 1, 2 or 3; and
the content of the active ingredient A is selected from 1.0%-90.0%, preferably 5.0%-75.0%, more preferably 10.0%-50.0%.

2. The pharmaceutical composition according to claim 1, **characterized in that** the pharmaceutical composition comprises 10-800 mg of the active ingredient A (calculated based on a free base).

3. The pharmaceutical composition according to claim 2, **characterized in that** the pharmaceutical composition comprises 10-400 mg of the active ingredient A (calculated based on a free base).

**4.** The pharmaceutical composition according to claim 3, **characterized in that** the pharmaceutical composition comprises 20-400 mg of the active ingredient A (calculated based on a free base).

**5.** The pharmaceutical composition according to claim 4, **characterized in that** the pharmaceutical composition comprises 30-200 mg of the active ingredient A (calculated based on a free base).

**6.** The pharmaceutical composition according to claim 2, **characterized in that** the pharmaceutical composition comprises 30 mg, 50 mg, 100 mg, 200 mg, 300 mg, 400 mg, 500 mg, 600 mg, 700 mg, or 800 mg of the active ingredient A (calculated based on a free base).

**7.** The pharmaceutical composition according to claim 1, **characterized in that** the pharmaceutical composition comprises the active ingredient A and a pharmaceutically acceptable excipient, the active ingredient A being selected from the compound represented by general formula (I) or a stereoisomer, a tautomer, a deuterated form, a solvate, a prodrug, a metabolite, a pharmaceutically acceptable salt or an eutectic crystal thereof, and the structure of the compound represented by general formula (I) being selected from one of the structures shown in Table S-1.

**8.** The pharmaceutical composition according to claim 1, **characterized in that** the structure of the compound represented by general formula (I) is selected from the following structure:

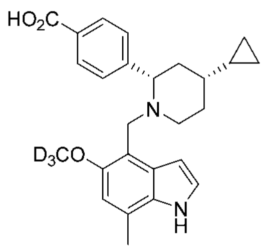

(Compound 1).

**9.** The pharmaceutical composition according to claim 1, **characterized in that** the active ingredient A is selected from compound 1 or succinate of compound 1, and preferably the molar ratio of compound 1 to succinic acid is 1 : 0.75.

**10.** The pharmaceutical composition according to claim 1, **characterized in that** the pharmaceutically acceptable excipient is selected from one or more of a filler, a disintegrant, a binder, a glidant and a lubricant.

**11.** The pharmaceutical composition according to claim 10, **characterized in that** the filler is selected from one or more of microcrystalline cellulose, mannitol, lactose, sucrose, sorbitol, dextran, pregelatinized starch, calcium dihydrogen phosphate and starch.

**12.** The pharmaceutical composition according to claim 11, **characterized in that** the content of the filler is selected from 10.0%-90.0%, preferably 20.0%-80.0%.

**13.** The pharmaceutical composition according to claim 11, **characterized in that** the filler is selected from a combination of microcrystalline cellulose and lactose, the content thereof being selected from10.0%-90.0%, preferably 20.0%-80.0%, and the content ratio of microcrystalline cellulose to lactose being 2 : 1 to 1 : 3, preferably 1 : 1 to 1 : 2.

**14.** The pharmaceutical composition according to claim 10, **characterized in that** the disintegrant is selected from one or more of sodium carboxymethyl starch, low-substituted hydroxypropyl cellulose, crospovidone, croscarmellose sodium and calcium carboxymethylcellulose, the content thereof being selected from 1.0%-10.0%, preferably 3.0%-8.0%.

**15.** The pharmaceutical composition according to claim 10, **characterized in that** the binder is selected from one or more of povidone, hydroxypropyl cellulose, hypromellose and methylcellulose, the content thereof being selected from 1.0%-10.0%, preferably 2.0%-8.0%.

**16.** The pharmaceutical composition according to claim 10, **characterized in that** the wetting agent is selected from one or both of water and ethanol.

17. The pharmaceutical composition according to claim 10, **characterized in that** the glidant is selected from one or more of talc, silica, colloidal silicon dioxide, polyethylene glycol and magnesium lauryl sulfate, the content thereof being selected from 0.1%-3.0%.

18. The pharmaceutical composition according to claim 10, **characterized in that** the lubricant is selected from one or more of magnesium stearate, calcium stearate, stearic acid and sodium stearyl fumarate, the content thereof being selected from 0.1%-3.0%.

19. The pharmaceutical composition according to claim 10, **characterized in that** the pharmaceutically acceptable excipient further comprises one or more of a flavoring agent, an antioxidant, a preservative, an opacifier and a film coating premixer.

20. Use of the pharmaceutical composition according to any one of claims 1-19 in the preparation of a related drug for treating nephropathy.

**INTERNATIONAL SEARCH REPORT**

International application No.
**PCT/CN2024/077932**

**A. CLASSIFICATION OF SUBJECT MATTER**

A61K 31/454(2006.01)i; C07D401/02(2006.01)i; C07D401/06(2006.01)i; C07D403/02 (2006.01)i; C07D403/06(2006.01)i; A61K9/00(2006.01)i; A61K47/00 (2006.01)i; A61P13/12(2006.01)i

According to International Patent Classification (IPC) or to both national classification and IPC

**B. FIELDS SEARCHED**

Minimum documentation searched (classification system followed by classification symbols)

IPC: A61K31/-, C07D401/-, C07D403/-, A61K9/-, A61K47/-, A61P13/-

Documentation searched other than minimum documentation to the extent that such documents are included in the fields searched

Electronic data base consulted during the international search (name of data base and, where practicable, search terms used)

CNTXT; VEN; ENTXT; WOTXT; USTXT; EPTXT; GBTXT; JPTXT; CNKI; 万方, WANFANG; 百度学术, BAIDU SCHOLAR; 读秀, DUXIU; Web of Science; STN: 西藏海思科制药有限公司, 张轩邈, 窦赢, 毛华, 邓士豪, 周洋, 苯丙氮杂芳, 药物组合物, 补体因子B, 肾脏, XIZANG HAISCO PHARMACEUTICAL CO., LTD., benzazaaryl, pharmaceutical composition, complement factor B, kidney, 通式I, 化合物1

**C. DOCUMENTS CONSIDERED TO BE RELEVANT**

| Category* | Citation of document, with indication, where appropriate, of the relevant passages | Relevant to claim No. |
|---|---|---|
| PX | WO 2023020566 A1 (SICHUAN HAISCO PHARMACEUTICAL CO., LTD.) 23 February 2023 (2023-02-23)<br>claims 1-20 | 1-20 |
| X | WO 2022256586 A2 (CHINOOK THERAPEUTICS, INC.) 08 December 2022 (2022-12-08)<br>description, paragraphs 8-10, 180, 307-308, 312-315, and 319, paragraph 181, componds 41 and 43, and paragraphs 930-934, embodiments 41 and 43 | 1-20 |
| Y | WO 2022256586 A2 (CHINOOK THERAPEUTICS, INC.) 08 December 2022 (2022-12-08)<br>description, paragraphs 8-10, 180, 307-308, 312-315, and 319, paragraph 181, componds 41 and 43, and paragraphs 930-934, embodiments 41 and 43 | 1 |
| Y | CN 114057692 A (SHANGHAI MEIYUE BIOTECH DEVELOPMENT CO., LTD.) 18 February 2022 (2022-02-18)<br>description, paragraphs 10, 175, 177, and 186 | 1 |
| X | WO 2022143940 A1 (MEDSHINE DISCOVERY INC.) 07 July 2022 (2022-07-07)<br>description, embodiments 1 and 3, and experiments 1-4 | 1-20 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 April 2024** | **06 May 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

INTERNATIONAL SEARCH REPORT

International application No.
PCT/CN2024/077932

C. DOCUMENTS CONSIDERED TO BE RELEVANT

| Category* | Citation of document, with indication, where appropriate, of the relevant passages | Relevant to claim No. |
|---|---|---|
| A | CN 105579444 A (NOVARTIS AG) 11 May 2016 (2016-05-11)<br>claims 1-25 | 1-20 |
| A | CN 109414441 A (ACHILLION PHARMACEUTICALS, INC.) 01 March 2019 (2019-03-01)<br>claims 1-23 | 1-20 |
| A | US 2021269416 A1 (NOVARTIS AG) 02 September 2021 (2021-09-02)<br>claims 1-27 | 1-20 |
| A | US 2016152605 A1 (ADAMS CHRISTOPHER et al.) 02 June 2016 (2016-06-02)<br>claims 1-21 | 1-20 |

Form PCT/ISA/210 (second sheet) (July 2022)

**INTERNATIONAL SEARCH REPORT**
**Information on patent family members**

International application No.
**PCT/CN2024/077932**

| Patent document cited in search report | Publication date (day/month/year) | Patent family member(s) | Publication date (day/month/year) |
|---|---|---|---|
| WO 2023020566 A1 | 23 February 2023 | CA 3229360 A1 | 23 February 2023 |
| | | TW 202321212 A | 01 June 2023 |
| | | AU 2022329230 A1 | 21 March 2024 |
| WO 2022256586 A2 | 08 December 2022 | CO 2023016557 A2 | 26 February 2024 |
| | | IL 308835 A | 01 January 2024 |
| | | CA 3222144 A1 | 08 December 2022 |
| | | EP 4346812 A2 | 10 April 2024 |
| | | AU 2022283903 A1 | 30 November 2023 |
| | | ECSP 23090260 A | 29 February 2024 |
| | | WO 2022256586 A3 | 12 January 2023 |
| | | DOP 2023000263 A | 29 February 2024 |
| | | BR 112023025235 A2 | 27 February 2024 |
| | | KR 20240017004 A | 06 February 2024 |
| CN 114057692 A | 18 February 2022 | WO 2022028507 A1 | 10 February 2022 |
| | | EP 4194449 A1 | 14 June 2023 |
| | | EP 4194449 A4 | 20 March 2024 |
| | | US 2023331710 A1 | 19 October 2023 |
| WO 2022143940 A1 | 07 July 2022 | AU 2021414253 A1 | 17 August 2023 |
| | | US 2024109861 A1 | 04 April 2024 |
| | | CA 3203447 A1 | 07 July 2022 |
| | | JP 2024501562 A | 12 January 2024 |
| | | EP 4273137 A1 | 08 November 2023 |
| CN 105579444 A | 11 May 2016 | IL 243540 A0 | 29 February 2016 |
| | | IL 243540 B | 26 September 2019 |
| | | PL 3022192 T3 | 30 March 2018 |
| | | DK 3022192 T3 | 22 January 2018 |
| | | MY 187454 A | 22 September 2021 |
| | | UA 117371 C2 | 25 July 2018 |
| | | JP 2016526576 A | 05 September 2016 |
| | | JP 6378761 B2 | 22 August 2018 |
| | | NZ 715780 A | 26 March 2021 |
| | | AP 201608992 D0 | 31 January 2016 |
| | | EA 201690223 A1 | 30 June 2016 |
| | | EA 031030 B1 | 30 November 2018 |
| | | AP 2016008992 A0 | 31 January 2016 |
| | | PH 12016500072 A1 | 18 April 2016 |
| | | JO 3425 B1 | 20 October 2019 |
| | | CU 20160006 A7 | 31 August 2016 |
| | | CU 24397 B1 | 04 April 2019 |
| | | PE 20161066 A1 | 30 October 2016 |
| | | US 2018009795 A1 | 11 January 2018 |
| | | US 10093663 B2 | 09 October 2018 |
| | | AU 2014290298 A1 | 11 February 2016 |
| | | AU 2014290298 B2 | 15 June 2017 |
| | | MX 351291 B | 09 October 2017 |
| | | CA 2917839 A1 | 22 January 2015 |
| | | CA 2917839 C | 03 May 2022 |
| | | SG 11201600086 PA | 26 February 2016 |
| | | HRP 20172000 T1 | 09 February 2018 |
| | | PT 3022192 T | 15 January 2018 |

Form PCT/ISA/210 (patent family annex) (July 2022)

**INTERNATIONAL SEARCH REPORT**
**Information on patent family members**

International application No.
**PCT/CN2024/077932**

| Patent document cited in search report | Publication date (day/month/year) | Patent family member(s) | Publication date (day/month/year) |
|---|---|---|---|
| | | TW 201546058 A | 16 December 2015 |
| | | TWI 641600 B | 21 November 2018 |
| | | CY 1119767 T1 | 27 June 2018 |
| | | HK 1221217 A1 | 26 May 2017 |
| | | LT 3022192 T | 27 December 2017 |
| | | SI 3022192 T1 | 31 January 2018 |
| | | UY 35663 A | 27 February 2015 |
| | | IL 268623 A | 31 October 2019 |
| | | IL 268623 B | 30 January 2020 |
| | | GT 201600007 A | 18 December 2018 |
| | | EP 3299365 A1 | 28 March 2018 |
| | | HUE 037510 T2 | 28 September 2018 |
| | | CL 2016000060 A1 | 10 June 2016 |
| | | WO 2015009616 A1 | 22 January 2015 |
| | | MY 196427 A | 10 April 2023 |
| | | TN 2016000017 A1 | 05 July 2017 |
| | | SV 2016005137 A | 12 June 2018 |
| | | BR 112016000909 A2 | 25 July 2017 |
| | | BR 112016000909 A8 | 07 January 2020 |
| | | BR 112016000909 B1 | 02 May 2023 |
| | | ES 2655855 T3 | 21 February 2018 |
| | | US 2016152605 A1 | 02 June 2016 |
| | | US 9682968 B2 | 20 June 2017 |
| | | KR 20160030556 A | 18 March 2016 |
| | | KR 102242742 B1 | 23 April 2021 |
| | | EP 3022192 A1 | 25 May 2016 |
| | | EP 3022192 B1 | 11 October 2017 |
| | | RS 56721 B1 | 30 March 2018 |
| CN 109414441 A | 01 March 2019 | WO 2018005552 A1 | 04 January 2018 |
| | | IL 263783 A | 31 January 2019 |
| | | ES 2902006 T3 | 24 March 2022 |
| | | US 2022119400 A1 | 21 April 2022 |
| | | EP 3939591 A1 | 19 January 2022 |
| | | MX 2018016046 A | 02 May 2019 |
| | | KR 20190036520 A | 04 April 2019 |
| | | JP 2019527203 A | 26 September 2019 |
| | | JP 7057290 B2 | 19 April 2022 |
| | | US 2019135825 A1 | 09 May 2019 |
| | | US 10961252 B2 | 30 March 2021 |
| | | JP 2022091984 A | 21 June 2022 |
| | | RU 2018145364 A | 28 July 2020 |
| | | EP 3448389 A1 | 06 March 2019 |
| | | EP 3448389 A4 | 30 October 2019 |
| | | EP 3448389 B1 | 29 September 2021 |
| | | US 2020165262 A1 | 28 May 2020 |
| | | US 11248000 B2 | 15 February 2022 |
| | | AU 2017290593 A1 | 03 January 2019 |
| | | SG 11201811491 YA | 30 January 2019 |
| | | CA 3029262 A1 | 04 January 2018 |
| US 2021269416 A1 | 02 September 2021 | IL 279992 A | 01 March 2021 |

Form PCT/ISA/210 (patent family annex) (July 2022)

**INTERNATIONAL SEARCH REPORT**
**Information on patent family members**

International application No.
**PCT/CN2024/077932**

| Patent document cited in search report | Publication date (day/month/year) | Patent family member(s) | Publication date (day/month/year) |
|---|---|---|---|
| | | JP 2021530505 A | 11 November 2021 |
| | | AU 2019304216 A1 | 21 January 2021 |
| | | AU 2019304216 B2 | 02 June 2022 |
| | | KR 20210032950 A | 25 March 2021 |
| | | US 11208398 B2 | 28 December 2021 |
| | | EP 3823963 A2 | 26 May 2021 |
| | | US 2022169630 A1 | 02 June 2022 |
| | | US 11578053 B2 | 14 February 2023 |
| | | WO 2020016749 A2 | 23 January 2020 |
| | | WO 2020016749 A3 | 05 March 2020 |
| | | CA 3106124 A1 | 23 January 2020 |
| US 2016152605 A1 | 02 June 2016 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2022113216 W
- WO 2015009616 A
- WO 2022028507 A

### Non-patent literature cited in the description

- Chinese Pharmacopoeia. 2020, vol. IV